(19) Europäisches Patentamt

European Patent Office

Office européen des brevets

(11) **EP 1 469 826 B1**

(12) **EUROPEAN PATENT SPECIFICATION**

(45) Date of publication and mention
of the grant of the patent:
**22.11.2006 Bulletin 2006/47**

(21) Application number: **03701640.9**

(22) Date of filing: **28.01.2003**

(51) Int Cl.:
***A61K 9/00*** (2006.01)

(86) International application number:
**PCT/IB2003/000265**

(87) International publication number:
**WO 2003/063823 (07.08.2003 Gazette 2003/32)**

(54) **OSMOTIC DELIVERY SYSTEM**

OSMOTISCHES VERABREICHUNGSSYSTEM

SYSTEME DE DISTRIBUTION OSMOTIQUE

(84) Designated Contracting States:
**AT BE BG CH CY CZ DE DK EE ES FI FR GB GR
HU IE IT LI LU MC NL PT SE SI SK TR**

(30) Priority: **01.02.2002 US 353151 P**

(43) Date of publication of application:
**27.10.2004 Bulletin 2004/44**

(73) Proprietor: **Pfizer Products Inc.
Groton,
Connecticut 06340 (US)**

(72) Inventors:
• **BILLOTE, Anne Martine
San Diego, CA 92121 (US)**
• **CARRIER, Rebecca Lyn
Groton, CT 06340 (US)**
• **FERGIONE, Michael Bruce
Groton, CT 06340 (US)**
• **MILLER, Lee Anthony
Groton, CT 06340 (US)**

• **ROY, Michael Christopher
Groton, CT 06340 (US)**
• **SHAMBLIN, Sheri Lynn
Groton, CT 06340 (US)**
• **WATERMAN, Kenneth Craig
Groton, CT 06340 (US)**
• **MACDONALD, Bruce Clinton
Groton, CT 06340 (US)**
• **FRIESEN, Dwayne Thomas
Bend, OR 97701 (US)**

(74) Representative: **Ruddock, Keith Stephen
Pfizer Limited
European Patents Department,
Ramsgate Road
Sandwich,
Kent CT13 9NJ (GB)**

(56) References cited:
**WO-A-01/91716          US-A- 5 543 155**

EP 1 469 826 B1

**Description**

FIELD OF INVENTION

The present invention relates to a pharmaceutical osmotic delivery system, in particular, a simple osmotic tablet for delivering low-solubility pharmaceutical agents.

BACKGROUND

The use of oral therapeutic systems having extended release of a drug for effecting a controlled systemic response over time and their advantages over conventional dosage forms such as dispersible tablets and syrups are well-known in the art. Of particular interest are the osmotic systems. The pioneering work for elementary osmotic pumps (also referred to as "simple osmotic systems") is described by Theeuwes in J. Pharm. Sc., **64**(12), 1987-1991 (1975), and in U.S. Patent Nos. 3,845,770; 3,916,899; 4,077,407; and 4,160,020. The osmotic dispensing device is based on an internal/external osmotic pressure differential (e.g., osmotic pressure gradient across a water-permeable wall against an external fluid). In the simple osmotic system, the device is in the form of a tablet consisting of a solid core surrounded by a water-permeable membrane. Aqueous body fluids enter the system continuously through the water-permeable membrane and dissolve the solid active substance contained within the core. The drug is then released through an orifice in the membrane once sufficient pressure is built up to cause the solution containing the drug to be pushed through the orifice. When the active substance present in the core is able to produce a sufficiently high osmotic pressure of its own or when additives are present to increase the osmotic pressure (i.e., osmagents), the drug is released at a predetermined rate to achieve the desired therapeutic effect. The prerequisite for achieving this effect is a sufficiently high solubility of water-soluble drug such that the amount of water entering the core through the water-permeable membrane is sufficient to dissolve most of the drug in the core. As a result, the drug is delivered from the tablet in a predominantly soluble form.

For drugs that are insoluble or have low-solubility in the fluid environment (e.g., bodily fluids), osmotically controlled delivery of the drug to elicit the desired therapeutic effect is more difficult. For this reason, the simple osmotic systems have been generally considered unsuitable for insoluble or low solubility drugs.

One approach for solving this problem is described in U.S. Patent No. 4,615,698 which discloses the use of a collapsable water-permeable wall that surrounds the pharmaceutical core. The drug or drug/osmagent may be present alone or in combination with a viscosity-inducing agent. The viscosity-inducing agent acts by increasing the viscosity surrounding the drug in the device and thereby entraining the drug in the exiting fluid.

Several different non-ionic water-soluble compounds are listed as suitable viscosity inducing agents. Unlike the earlier osmotic devices, the water-permeable wall collapses as the drug is delivered through an orifice in the wall. The advantage of this system is the nearly complete delivery of low solubility drug from the device. However, to function properly, the outer membrane must be designed such that it does not rupture from the osmotic pressure generated within the core. As a result, finding the proper thickness and elasticity of the membrane for a particular application and then maintaining those properties during manufacture of the device can be difficult. To date, no commercial embodiment using this technology has been realized.

Another approach involves two-compartment systems (also known as "push-pull" systems). See, e.g., U.S. Patent No. 4,111,202. In a push-pull system, the drug or drug formulation is present in one compartment and water-soluble or water-swellable auxiliaries (e.g. salts, sugars, swellable polymers and hydrogels) for producing an osmotic pressure are present in a second compartment. The two compartments are separated from each other by a flexible partition and sealed externally by a rigid water-permeable membrane. Fluids entering the second compartment cause an increase in volume of the first compartment, which in turn acts on the expanding flexible partition and expels the contents of the drug compartment from the system. The preparation of push-pull systems is technically complicated. For example, a flexible partition consisting of a material different from that of the water-permeable membrane has to be incorporated into the dosage form. In addition, for sparingly soluble high-dosage drugs (e.g. more than 200 mg dose), a push-pull system would be voluminous, thus making its ingestion difficult.

Push-pull systems for sparingly soluble drugs without a partition are disclosed in U.S. Patent No. 4,327,725. A commercial embodiment of this system is known as GITS (gastro-intestinal therapeutic system) and is marketed in commercial products such as Procardia™ XL and Glucotrol™ XL (both available from Pfizer, Inc., New York, NY). The core consists of two layers: one layer containing the drug and a second layer containing an osmotic driving member. A rigid water-permeable layer surrounds the core and contains a passageway in communication with the drug layer only. The osmotic driving member is a swellable polymer or hydrogel (e.g., polyethylene oxide). Absorption of fluid into the system causes the hydrogel in the second layer to expand thus forcing the contents of the drug layer through the passageway. Compared with conventional coated tablets, the preparation of these tablets is complicated. Not only does this system require a more complex bilayer press to tablet, but also, stringent demands are placed on the properties of the two formulations being compressed together to form a cohesive core. In addition, placement of the passageway is critical to the successful delivery of the drug (e.g., the orifice must be in communication with only the drug containing layer). This system is generally limited to doses of active drug or combination of drug and functional additives lower than about 100 mg.

Another approach for delivering sparingly soluble drugs in an osmotic tablet is the addition of a gas generating means to the tablet core. U.S. Patent Nos. 4,036,228 and 4,265,874 disclose a single layer core containing a limited solubility drug, a gas generating means (e.g., effervescent couple), an osmagent and a surfactant having wetting, solubilizing and foaming properties (e.g., sodium lauryl sulfate). Fluids imbibing through a rigid water-permeable membrane surrounding the core cause the gas-generating means to produce a gas which creates a pressure sufficient to expel the drug through an orifice in the membrane. Providing a sufficient pressure to expel a low solubility drug over an extended period has proved challenging, such that this technology has not been commercialized.

US Patent numbers 4,627,850 and 5,869,097 disclose the use of osmotic caplets whereby the caplets are formed by coating of capsules with semipermeable membranes. This process is cumbersome and difficult to manufacture. In addition, for low solubility drugs, the system requires a separate compartment containing an osmopolymer designed to swell and thereby expel the drug. Such a system is complex and cannot provide for a high dose of drug in a form ingestible by a subject.

Numerous patents have issued which focus on increasing the solubility of specific sparingly soluble drugs in an osmotic system. For example, U.S. Patent Nos. 4,610,686 and 4,732,915 disclose the addition of organic acids to increase the solubility of Haloperidol and U.S. Patent No. 6,224,907B1 discloses the addition of an alkalinizing agent as a solubility enhancer for a leukatriene-receptor antagonist. The success of this approach in enabling elementary osmotic pump type systems is dependent upon the basicity or acidity of the drug being delivered and the solubility achieved. For many drugs, solubilizing strategies will still not allow sufficient solubility to enable elementary osmotic pumps, or will cause other complications. For example, solubilizing additives will lead to more material in the drug core thereby reducing the amount of drug deliverable by this technology. In other cases, solubilizing additives may adversely affect the drug's stability.

US Patent No. 4,857,336 (Re. 34,990) discloses an oral therapeutic osmotic system for carbamazepine having only one drug compartment. Although carbamazepine has a low solubility in water, the primary problem being addressed was crystal growth (i.e., ripening) of carbamezepine upon storage or when the water encounters the drug. According to the disclosure, the crystal growth of carbamazepine can be inhibited by adding a protective colloid (e.g., hydroxypropylmethylcellulose) to the drug formulation in the core. An improvement of this formulation is disclosed in U.S. Patent 5,284,662 which utilizes a mixture of two different hydroxy ($C_1$-$C_4$)alkyl celluloses in combination with the crystal habit modifier and a 1:9 to 9:1 ratio of a $C_6$-sugar and a mono- or di-saccharide to improve the delivery of carbamazepine from the device.

WO 01 91716 A discloses a therapeutic system for peroral administration comprising (i) a single layer core of glipizide, vinylpyrrolidone/vinyl acetate copolymer and ethylene oxide homopolymer (PEO), (ii) a semi-permeable wall and (iii) a passageway.

US-A-5 543 155 discloses a diffusion-osmotic controlled drug release pharmaceutical composition comprising (i) a layer core containing a therapeutically active agent and a hydrophilic polymer, (ii) a polymeric film-coat coating said tablet core and (iii) at least one bore in said film-coat in contact with said tablet core.

Other single-layer osmotic tablets have also been reported. For example Andrx Pharmaceuticals has reported use of single-layer osmotic systems for delivery of highly-water soluble or low dose drugs as disclosed in United States Patents 5,654,005; 5,736,159; 5,837,379; 6,099,859; and 6,156,342. Shire Laboratories discloses in United States Patent 6,110,498 the use of unitary osmotic cores to deliver drugs in soluble form.

For reviews that summarize the patent literature and compare the various approaches used in osmotic systems, see Verma, R.K., et al., Drug Development and Industrial Pharmacy, **2617**, 695-708 (2000) and Santus, G., et al., "Osmotic drug delivery: a review of the patent literature," Journal of Controlled Release, **35,** 1-21 (1995).

When delivering a high dose of a water-insoluble drug, it is desirable to provide that dosage form in a shape that facilitates swallowing. For example, it would be advantageous to provide an osmotic dosage form in the shape of a caplet or an oblong shape. In bilayer osmotic drug delivery systems, manufacturing by standard bilayer tablet presses requires that the die and punch have the caplet or oblong shape to produce the desired tablets. This necessitates filling the lower die with "push" layer at a greater width than with a more symmetrical shape. Since the fill is height-controlled, the push-layer will have more mass than for a corresponding symmetrical shape. Moreover, bilayer osmotic tablets suitable for low solubility drugs produce significant strain on the coating as they swell during use. This tendency to split the coating open and potentially dump a drug in an uncontrolled manner favors a more symmetrical tablet shape. As such, bilayer osmotic tablets have always been produced commercially in a standard tablet shape (usually, standard round concave, SRC). It therefore is also an aim of the present invention to provide an osmotic drug delivery system suitable for low solubility drugs that can function with shapes more easily swallowed.

Although several different approaches have been tried in an attempt to incorporate insoluble or low-solubility drugs into an effective osmotic system, there still remains a need for improved systems that provide a more predictable formulation for a wider variety of drug classes and a convenient means for manufacture. In particular, the need remains to provide an improved system capable of delivering higher drug doses of low solubility drugs in a convenient overall dosage size.

SUMMARY

An osmotic pharmaceutical tablet is provided which comprises (a) a single-layer compressed core comprising: (i) a non-ripening drug having a solubility per dose less than about 1 mL$^{-1}$, (ii) a hydroxyethylcellulose having a weight-average, molecular weight from about 300,000 to about 2,000,000 (preferably between about 700,000 and 1,500,000), and (iii) an osmagent, wherein the hydroxyethylcellulose is present in the core from about 2.0% to about 20% by weight (preferably from about 3% to about 15%, more preferably from about 5% to about 10%, and the osmagent is present from about 15% to about 75% by weight (preferably from about 20% to about 75%, more preferably from about 40% to about 60%, most preferably from about 40% to about 55%); (b) a water-permeable layer surrounding the core; and (c) at least one passageway within the layer (b) for delivering the drug to a fluid environment surrounding the tablet. In a preferred embodiment, the tablet is shaped such the surface area to volume ratio (of a water-swollen tablet) is greater than 0.6 mm$^{-1}$; more preferably greater than 1.0 mm$^{-1}$. It is preferred that the passageway connecting said core with the fluid environment be situated along the tablet band area, as more fully explained hereinafter. A particularly preferred shape is an oblong shape where the ratio of the tablet tooling axes, i.e., the major and minor axes which define the shape of the tablet, are between 1.3 and 3; more preferably between 1.5 and 2.5. In another preferred embodiment, the combination of the non-ripening drug and the osmagent have a weight average ductility from about 100 to about 200 Mpa, a weight average tensile strength from about 0.8 to about 2.0 Mpa, and a weight average brittle fracture index less than about 0.2.

An osmotic dosage form according to the invention comprises a non-ripening drug and a pharmaceutically acceptable carrier. The drug preferably constitutes at least 30% by weight of the core, based on the weight of the core. The carrier is conceptually thought of as comprising the accessory, non-drug excipients also used in the dosage form. Conventional excipients comprising the carrier may include, *inter alia,* binders, diluents, flavorings, buffers, colors, lubricants, thickening agents, and the like. Some excipients can serve multiple functions, for example as both binder and diluent. Necessary excipients in this invention include osmagents and a high molecular weight hydroxyethylcellulose. Some preferred excipients in this invention include bioavailability enhancers (including acids for pH control or adjustment), cyclodextrins (preferably β-cyclodextrin or sulfobutyl-β-cyclodextrin), dispersing aids, and lubricants. The single-layer core may, for example, optionally include a bioavailability enhancing additive, and/or other pharmaceutically acceptable excipients, or diluents. The non-ripening drug may be a non-crystalline drug, a crystalline drug, or a drug particle comprising a non-crystalline or crystalline drug and an excipient.

### *Definitions*

As used herein, the term "non-ripening" is defined as those pharmaceutical agents that are either (i) a non-crystalline drug form (e.g., amorphous drug or drug-excipient solid solution), or (ii) a crystalline drug form (e.g., polymorphic or hydrate form) having an average particle size in the dosage form such that the average particle size does not increase significantly in size upon contact with moisture (either from storage under normal storage conditions or during operation of the device) in the absence of a protective colloid or crystal-habit modifier. The term "crystal-habit modifier" or "protective colloid" refers to excipients either in a separate phase from the drug particles (powder mixture) or adsorbed to the particle surface which function to prevent crystal growth. A "significant particle size change" is one that hinders the performance of the drug *in vivo,* for example, by affecting its dissolution rate or ability to be delivered from the dosage form resulting in an at least about 20% decrease in bioavailability, as indicated.

An example of the crystal growth (ripening) process with pharmaceutical solids can be found in H. Weiss, Pharmazie, **32**, 624-625 (1977). For comparative purposes, an example of a "ripening" drug is anhydrous carbamazepine which grows long needles when contacted with moisture (as it forms the dihydrate). In the case of anhydrous carbamazepine, ripening in the absence of stabilizing colloids hinders both its absorption *in vivo* and its delivery from an osmotic device

The term "limited-solubility" refers to those pharmaceutical agents having a solubility less than about 40 mg/mL at a physiologically relevant pH (e.g., pH 1-8). Included within the meaning of limited-solubility are those drugs that are "low solubility" (defined herein as solubility less than 2 mg/mL), "substantially water-insoluble" (defined herein as a drug having a water solubility of less than about 10 μg/mL at a physiologically relevant pH), "sparingly water-soluble" (defined herein as a drug having an aqueous solubility of about 10 micrograms/mL up to about 1 to 2 mg/mL), and "moderately soluble" (defined herein as a drug having an aqueous solubility as high as about 2 to 40 mg/mL).

The term "limited-solubility per dose" refers to active pharmaceutical agents having solubilities divided by their doses of less than about 1 mL$^{-1}$.

The term "osmagent" or "osmotic agent" refers to any agent that creates a driving force for transport of water from the environment of use into the core of the osmotic device.

The term "drug" refers to a pharmaceutically active ingredient(s) including any of its conventional pharmaceutical forms, including a pharmaceutically acceptable salt thereof, a solvate (including hydrate) of the active ingredient or salt, or a prodrug of the active ingredient, salt or solvate and any pharmaceutical composition formulated to elicit a therapeutic effect in a human or animal. In cases where the drug solubility is sufficiently high, the drug can also act as an osmagent, thereby reducing the amount of excipient needed to deliver a given amount of drug.

The term "bioavailability enhancing additive" or "bioavailability enhancer" refers to an additive known in the art to increase bioavailability (e.g., solubilizing agents, additives that increase drug permeability in the GI tract, enzyme inhibitors, and the like). A bioavailability enhancing additive can be identified by determining the pharmacokinetics of formulations with and without the enhancer. The enhancer should show a mean AUC (area under the curve) increase of at least 25% relative to that without the enhancer.

## BRIEF DESCRIPTION OF THE DRAWINGS

In the drawings, which are not drawn to scale, three modes of preferred tablet shapes are shown. Each succeeding pair of Figures (i.e., Figures 1-2, Figures 3-4, and Figures 5-6) illustrates a preferred shape.

FIG. 1 is a side view of a preferred oblong shaped tablet with an orifice shown in a preferred position.

FIG. 2 is a top view of the tablet of Figure 1.

FIG. 3 is a side view of a caplet-shaped tablet with an orifice shown in a preferred position.

FIG. 4 is a top view of the caplet-shaped tablet of Figure 3.

FIG. 5 is a side view of a preferred lozenge-shaped tablet having inwardly extending opposing faces.

FIG. 6 is a top view of the lozenge-shaped tablet of Figure 5 with an orifice indicated in a preferred position.

FIG. 7 is a side view of an oblong tablet as shown in FIGURE 1, but wherein the tablet does not have a side band 3 shown in FIGURE 1.

## DETAILED DESCRIPTION OF THE INVENTION

The present invention provides an osmotic pharmaceutical tablet that is capable of delivering limited-solubility drugs without the aid of a separate swellable layer or compartment to force the drug from the device. In addition, the present invention allows one to formulate a dosage form to give a high dose for limited solubility drugs that is relatively small in size for easy ingestion and allows for the formation of shapes that make swallowing higher doses possible. Such a system is preferably in the form of an oblong shape with a hole on one end of the tablet. In its simplest form, the osmotic tablet of the present invention comprises a pharmaceutical single-layer core surrounded by a water-permeable coating having a passageway for delivery of the drug from the core.

The pharmaceutical core comprises a non-ripening drug having a limited solubility per dose (i.e., solubility/dose is less than about 1 mL$^{-1}$), an osmagent, and the water-soluble polymer hydroxyethylcellulose (HEC) having a weight-average molecular weight from about 300,000 to about 2,000,000; more preferably from about 700,000 to 1,500,000.

### Pharmaceutical Agent or Drug

Any non-ripening drug form may be used in the present invention; however, limited-solubility drugs (i.e., drugs having an aqueous solubility of less than about 40 mg/mL in the fluids imbibed into the osmotic device) that need to be delivered in a high dose are of particular interest; still more preferred are drugs that have water solubilities below about 20 mg/mL. The fluid environment is primarily intended to be the gastrointestinal tract, but could include other biological environments where a therapeutic agent can be used for human or animal treatment. Current technologies can accommodate low solubility drugs if the dose is sufficiently low; however, for high doses, there are currently only a limited number of technologies available. Applicants have determined that for a solubility-per-dose less than about 1 mL$^{-1}$, preferably less than 0.1 1mL$^{-1}$, more preferably less than 0.01 mL$^{-1}$, the present invention provides a beneficial osmotic drug delivery system. When it is desirable to deliver bioavailability enhancers in conjunction with the drug, the present invention enables the delivery of a significant amount of such additives such that the total amount of the drug plus bioavailability enhancing additives can be as high as about 750 mg (preferably less than about 650 mg).

Virtually any pharmaceutical agent having a solubility/dose less than about 1 mL$^{-1}$ may be used in the present invention. In addition, the drug may be employed in the form of its pharmaceutically acceptable salt as well as in its anhydrous, hydrated, and solvated form and/or in the form of a prodrug. As discussed above, the drug (in the form used) does not ripen in the osmotic device upon contact with moisture (e.g., moisture contact from storage or in operation).

The drug can also include a combination of active agents that act either independently or synergistically to provide one or more therapeutic benefits. It may be desirable to combine the drug with bioavailability enhancing additives that serve to improve the overall effectiveness of the active pharmaceutical agent(s). Suitable bioavailability enhancing additives include solubilizing agents which can increase the drug solubility in the biological environment, materials capable of sustaining supersaturation within the biological environment, pH modifiers, buffers, enzyme inhibitors, permeation enhancers, and the like.

As discussed earlier, a non-ripening drug is defined as those pharmaceutical agents that are either (i) non-crystalline drugs, or (ii) crystalline drugs that do not increase significantly in particle size upon exposure to moisture in the absence of a crystal-habit modifier or protective colloid. A significant particle size change is one that decreases the bioavailability (as indicated by the area under the curve (AUC) in a pharmacokinetic plot) of the drug more than about 20% *in vivo.* An example of the crystal growth (ripening) process with pharmaceutical solids may be found in H. Weiss, Pharmazie, **32**, 624-625 (1977). The pharmaceutical agent(s) may be crystalline, non-crystalline, or a mixture thereof so long as the particle size does not increase significantly in the dosage form such that the performance of the drug is hindered *in vivo.*

For comparative purposes, an example of a "ripening" drug is carbamazepine. The tendency for crystals to grow upon storage or in the presence of water is typically a property of both the chemical nature of the compound and its particle size. In general, the tendency for crystal growth is inversely proportional to the particle size (i.e., the smaller the particle, the higher the tendency for crystal growth upon exposure to moisture). Crystal growth can be especially troublesome under conditions where supersaturation temporarily occurs within the dosage form. For example, an anhydrous form of a drug may dissolve and then supersaturate the solution with respect to a more stable hydrate (e.g., small particles of anhydrous crystals of carbamezipine). Without protective colloids, the anhydrous crystals dissolve in water then ripen as large hydrated crystals (see, e.g., US. Patent No. 4,857,336). Another example is when the crystal size is below about 1 $\mu$m in diameter. A crystal habit modifier (protective colloid) is typically added which functions by changing the surface properties of the drug particles.

Unlike the crystalline ripening drugs described above, one of the drug forms suitable for use in the practice of the present invention is a homogeneous, non-crystalline mixture of the drug and excipients where the combination can supersaturate. Since the drug form is not crystalline, additives to sustain supersaturation do not function as protective colloids, and therefore fall within the scope of the present invention. An example of such a drug form is described in U.S. Pub. No.2002/0009494 A1.

A change in drug absorption with crystal size generally depends on the drug solubility, dose, and permeability through the GI walls. Suitable crystal sizes of the drug generally depend on the size of the passageway(s) present in the osmotic device and to some extent on the tendency for the particles to settle inside the dosage form during operation. Preferably, the average drug particle size in the practice of the present invention remains below about 500 $\mu$m, more preferably below about 300 $\mu$m, and most preferably less than about 200 $\mu$m, As discussed above, if the drug is crystalline, then the particle size is preferably greater than about 1 $\mu$m to avoid the need to add a crystal-habit modifier. For a detailed discussion of the effects of particle size on drug dissolution and oral drug absorption see R. J. Hintz and K. C. Johnson, Inter. J. Pharm. **51**, 9-17 (1989).

The non-ripening drug can be in any solid form (e.g., crystalline, amorphous, or mixtures thereof). The solid form may also include an excipient as part of the drug particles themselves. Drug-excipient combinations can be prepared by methods such as spray-drying, extrusion, lyophilization or other techniques known by those skilled in the art.

For the drug to be entrained in the extruding fluid as it exits the tablet, settling due to gravity or other forces should be avoided. Both the absolute particle density (versus the density of the entraining medium) and the particle size can affect entrainment and can therefore influence the residual level of drug remaining inside the water-permeable coating (or layer) after 24 hours. For that reason, in some cases, it is preferable to use smaller particle sizes (e.g., less than about 20 $\mu$m in diameter) to improve performance. Particle size reduction can be carried out as is known in the art using such micronizing methods as jet milling and rapid precipitation. For the practice of the present invention, it has been determined that good drug delivery is possible for particle sizes from below 2 $\mu$m to about 300 $\mu$m, mean diameters.

A preferred drug form is prepared with a process and formulation designed to supersaturate the drug in the use environment. Still more preferred is a drug form designed to maintain supersaturation for a sufficient amount of time in the use environment to allow absorption. For example, a drug that is co-administered with an enteric polymer as described in WO 0147495 A1, EP 1027886 A2, EP 1027885 A2, and U.S. Pub. No.2002/0009494 A1.

Those skilled in the art will recognize that the present invention is generally applicable to a wide range of therapeutic indications and drug classes. Preferred classes of drugs include antihypertensives, antianxiety agents, antidepressants, barbituates, anticlotting agents, anticonvulsants, blood glucose-lowering agents, decongestants, antihistamines, antitussives, antineoplastics, antiarrhythmic agents (such as $\beta$-blockers, calcium channel blockers and digoxin), anti-inflammatories, antipsychotic agents, cognitive enhancers, cholesterol-reducing agents, antiobesity agents, autoimmune disorder agents, anti-impotence agents, antibacterial and antifungal agents, hypnotic agents, anti-Parkinsonism agents, anti-Alzheimer's Disease agents, antibiotics, antiviral agents, and HIV protease inhibitors.

Examples of the above and other classes of drugs and therapeutic agents deliverable by the invention include the following: nifedipine, a suitable anti-impotence agent selected from the class of cGMP $PDE_V$ inhibitors, an example of which is sildenafil, including its pharmaceutically acceptable salts such as sildenafil citrate and sildenafil mesylate; sertraline, [3,6-dimethyl-2-(2,4,6-trimethyl-phenoxy)-pyridin-4-yl]-(1-ethylpropyl)-amine and 3,5-dimethyl-4-(3'-pentoxy)-2-(2',4',6'-trimethylphenoxy)pyridine; and ziprasidone, including its pharmaceutically acceptable salts

The active pharmaceutical agent is typically present in the core in an amount from about 1 to about 80% by weight, more preferably in an amount from about 2 to about 60%. The present invention is particularly suited to deliver high doses, preferably between 100 mgA and 600 mgA.

*Bioavailability Enhancing Additives*

Bioavailability enhancing additives include additives known in the art to increase bioavailability, such as solubilizing agents, additives that increase drug permeability in the GI tract, enzyme inhibitors, and the like. Suitable solubilizing additives include cyclodextrins and surfactants. Other additives that function to increase solubility include acidic or basic additives which solubilize a drug by changing the local pH in the GI tract to a pH where the drug solubility is greater than in the native system. Preferred additives are acids that function to both improve the drug solubility *in vivo* and to increase the osmotic pressure within the dosage form thereby reducing or eliminating the need for additional osmagents. Preferred acids include ascorbic acid, 2-benzenecarboxylic acid, benzoic acid, fumaric acid, citric acid, edetic acid, malic acid, sebacic acid, sorbic acid, adipic acid, glutamic acid, toluene sulfonic acid, and tartaric acid. A preferred subgroup for use in combination with non-ripening basic drugs consists of tartaric acid, adipic acid, ascorbic acid, benzoic acid, citric acid, fumaric acid, glutamic acid, malic acid, sorbic acid and toluene sulfonic acid. Bioavailability enhancing additives also include materials that inhibit enzymes that either degrade drug or slow absorption by, for example, effecting an efflux mechanism. Another group of bioavailability enhancing additives include materials that enable drug supersaturation in the GI tract. Such additives include enteric polymers as disclosed in Patents WO 0147495 A1, EP 1027886 A2 and EP 1027885 A2. Particularly preferred polymers of this type include hydroxypropylmethylcellulose acetate succinate (HMPCAS) and cellulose acetate phthalate (CAP).

Acids or bases can also function to mediate the pH within the dosage form core during use and thereby reduce the drug delivery sensitivity to the pH of the use environment. In particular, it has been observed that for some drugs, their dispersability depends on the pH of the dispersing water. For the dosage form of the present invention to function effectively, the drug must disperse, and thereby be entrained in the exiting fluid. For such drugs that have pH sensitivity in their dispersability, it has been determined that the addition of 5-25% by weight of a soluble acid or base (depending on the pH for optimal dispersability of the drug) allows for drug delivery to be essentially independent of the external environmental pH. A particularly preferred acid useful for basic drugs is tartaric acid. Preferred bases include alkaline metal and alkaline earth salts of carbonate, bicarbonate and oxide, sodium phosphate (dibasic and monobasic), triazine base, guanidine and N-methyl glucamine.

Because the osmotic tablet of the present invention enables a large amount of active material to be delivered in a relatively small dosage form (up to about 80% active compound plus performance improving excipients), it is particularly suited for delivery of bioavailability enhancing additives to improve drug performance *in vivo.*

*HYDROXYETHYLCELLULOSE*

Although several polymers have been disclosed in the art for use in osmotic tablets, Applicants have determined that only a small subset of those polymers provides a commercially useful means for drug delivery in a single-layer osmotic system suitable for limited-solubility drugs. Water-soluble polymers are added to keep drug particles suspended inside the dosage form before they exit through the passageway(s) (e.g., an orifice). High viscosity polymers (i.e., having molecular weights up to about 2,000,000) are useful in preventing settling. However, the polymer in combination with the drug is extruded through the passageway(s) under relatively low pressures. At a given extrusion pressure, the extrusion rate typically slows with increased viscosity. Applicants have surprisingly determined that high molecular weight hydroxyethylcellulose (HEC) in combination with the drug particles form high viscosity solutions with water but are still capable of being extruded from the tablets with a relatively low force. In contrast, other polymers and HECs (see Examples 2 and 3) having a low weight-average, molecular weight (i.e., less than about 300,000) do not form sufficiently viscous solutions inside the tablet core to allow complete delivery due to drug settling. Settling of the drug is a problem when tablets are prepared with no polymer added, which leads to poor drug delivery unless the tablet is constantly agitated to keep drug particles from settling inside the core. Settling is also problematic when the drug particles are large and/or of high density such that the rate of settling increases. An example of an HEC capable of forming solutions having a high viscosity yet still extrudable at low pressures is Natrosol™ 250H (high molecular weight hydroxyethylcellulose, available from Hercules Incorporated, Aqualon Division, Wilmington, DE; MW equal to about 1M and a degree of polymerization equal to about 3,700). Natrosol™ 250H provides effective drug delivery at concentrations as low as about 3% by weight of the core when combined with an osmagent (see Example 1 in the Examples section). Natrosol™ 250H NF is a high-viscosity grade nonionic cellulose ether which is soluble in hot or cold water. The viscosity of a 1% solution of Natrosol™ 250H using a Brookfield LVT (30 rpm) at 25°C is between about 1,500 and about 2,500 cps. With Natrasol™ 250G (having a weight-average molecular weight of 300,000), effective delivery of drug particles requires polymer concentrations greater than 9% by weight. With lower molecular weight HECs (e.g., Natrosol™ 250L) effective drug delivery is not achieved at up to 20% by weight polymer (see Example 4).

In Examples 2 and 3 of the Examples section, a comparison of the efficiency of drug delivery is made among a number of water-soluble polymers that are commonly used in osmotic devices (e.g., hydroxypropylmethylcellulose (HPMC), hydroxypropylcellulose (HPC), and Carbopol™ (acrylic acid homopolymer available from BF Goodrich, Cleveland, OH). Unlike the more commonly used water-soluble polymers, the Natrosol™ 250H provided 90% delivery of the drug in 24 hours under a standard test condition. In example 3, a comparison is made between high molecular weight HEC and poly(ethylene oxide) (Polyox WSR N-80 and coagulant grade, both available form Union Carbide Corp.) using a different drug. Thus HEC provides the unique properties to provide entrainment and also is easily extracted allowing more complete delivery of drug from the core to the surrounding fluid.

Preferred hydroxyethylcellulose polymers for use in the present invention have a weight-average, molecular weight from about 300,000 to about 2,000,000 and a degree of polymerization from about 1,500 to about 6,700; more preferred from 700,000 to 1,500,000 (degree of polymerization 3,500 to 5,000). Example 4 shows a comparison of low and high molecular weight HEC. The hydroxyethylcellulose polymer, when used at a molecular weight of 700,000 to 1,500,000, is typically present in the core in an amount from about 2.0% to about 20% by weight, preferably from about 3% to about 15%, more preferably from about 5% to about 10%. When the HEC is used in the 300,000 to 700,000 molecular weight range, the polymer is preferably present between 9 and 20%.

The HEC can also be in a form designed to retard gel formation and thereby allow more uniform dissolution. An example of such an HEC is the surface modified version of HEC (HEC-R) produced by Hercules Corp.

*Osmagent*

The core of the drug delivery device of the present invention includes an osmagent (or osmotic agent). The osmagent provides the driving force for transport of water from the environment of use into the core of the device. The osmagent is generally present in the core at a concentration from about 15% to about 75% by weight, preferably from about 25% to about 75%, more preferably from about 35% to about 60%, still more preferably from 40% to about 55%. A wide variety of osmagents can provide the osmotic pressure needed to drive the drug from the osmotic device. In general, drug delivery from the device is fairly independent of the specific osmagent used. This is shown in Example 6 in the Examples section. The following factors have proven to be useful in selecting an osmagent appropriate for use in the present invention:

(1) potential reaction of the osmagent and any osmagent impurities with the drug;
(2) effect of the osmagent on the solubility of the drug in the use environment;
(3) impact of the osmagent solubility on the drug delivery rate; and
(4) mechanical properties of the osmagent.

Preferably, the osmagent does not significantly lower the solubility of the drug in the use environment. This is particularly an issue when the osmagent is a salt. In many cases, salts can depress the solubility of a salt form of a drug by a common ion effect.

Since the osmagent is typically the bulk excipient, the tableting properties of the osmagent are also considered. Typical tableting properties include flow (generally for direct compressed tablets) and mechanical properties. For the practice of the present invention, it has been determined that the optimum choice of osmagent can be accomplished by matching the ductility, tensile strength and brittle fracture index (BFI) (described in Hiestand and Smith in Powder Technology, **38**, 145 (1984)) of potential osmagents with the material properties of the drug. For some drugs, the binding of the drug to itself is sufficiently high that the osmagent serves to prevent the drug crystals from forming hard granules (during granulation), in which case, using fine grain osmagents is preferred. When the drug mechanical properties are combined with those of the osmagent and any other excipients, the resulting total blend properties determine the ability to form tablets with the blend. If the particle sizes of the drug, the osmagent(s), and other excipients are comparable (within about 25%) the blend properties will be a weighted average of the components. For a first approximation, the properties of the average should preferentially fall within the following ranges to achieve good tablets (i.e., tablets with low friability): ductility from about 100 to about 200 MPa; tensile strength from about 0.8 to about 2.0 MPa; and brittle fracture index (BFI) less than about 0.2. As mentioned above, these properties refer to a blend of the drug, the osmagent (s), and other excipients wherein the particle sizes for each of these components are comparable. In some cases, a binder may be desired to improve the binding properties of the tablet. Suitable binders include hydroxypropylcellulose such as Klucel™ EXF (available from Hercules Incorporated, Aqualon Division, Wilmington, DE) and hydroxypropylmethylcellulose such as Pharmacoat™ 603 (available from Shin-Etsu Chemical Company, Japan).

Osmagents of different dissolution rates can sometimes be employed to influence how rapidly drug is initially delivered from the dosage form. For example, amorphous sugars such as Mannogem EZ and Pharmaburst (both available from SPI Pharma, Lewes, Delaware) can provide faster delivery during the first couple of hours the dosage form is in an aqueous environment.

In some cases, the osmagent can serve as a bioavailability enhancing additive. For example, some acids can solubilize some drugs in the GI tract as well as provide sufficient osmotic pressure for operation of the device. When this is possible, use of an osmagent as a solublizer (bioavailability enhancing additive) may be preferred since this allows for a maximum dose of active for a given tablet size. An example for the use of a solubilizing acid as an osmagent is illustrated in Example 8 of the Examples section below.

Preferred osmagents include salts, acids and sugars. Preferred salts include sodium chloride and potassium chloride. Preferred acids include ascorbic acid, 2-benzene carboxylic acid, benzoic acid, fumaric acid, citric acid, maleic acid, serbacic acid, sorbic acid, edipic acid, edetic acid, glutamic acid, toluene sulfonic acid and tartaric acid. A particularly preferred acid is tartaric acid. Preferred sugars include mannitol, sucrose, sorbitol, xylitol, lactose, dextrose and trehalose. A particularly preferred sugar is sorbitol. These osmagents can be used alone or as a combination of two or more osmagents.

Sugars are preferred herein as osmagents. A particularly preferred osmagent is sorbitol. Sorbitol can be used as direct compress excipient (as with Neosorb 30/60 DC available from Rouquette America, Inc. in Keokuk, IA) or in a smaller particle size suitable for use with granulations (such as Neosorb P110, available from the same vendor).

### Dispersing Aids

In the course of developing this dosage form, it was determined that for some drugs, the drug delivery was affected by the dissolution medium used for testing. More specifically, for some drugs, the pH of the dissolution medium affected the dosage form performance. This was traced to the ability of the drug to be dispersed in that medium. As such, it has been determined that certain additives to the dosage form can improve the dispersability of the drug in some dissolution media. Examples include dispersing aids (typically low weight-average molecular weight polar polymers such as carbomers or poly(vinylalcohols)), surfactants (such as sodium dodecylsulfate) or agents designed to make the pH inside the tablet core independent of the dissolution medium. A preferred example of the latter is to add an acidifying agent such as tartaric acid. When used, the acid is preferably at a level between 5-25% of the core components; more preferably between 10-20% of the core. Another preferred dispersing aid is a poloxamer, i.e., a block copolymer of polyethylene oxide and polypropylene oxide as disclosed in "Handbook Of Pharmaceutical Excipients", 3rd Edition, (American Pharmaceutical Association) 2000, pp.386-388. When used in the present invention, it has been found that the poloxamer is most effective when in intimate contact with the drug. Such intimate contact can be achieved by, for example, coating a solution of the poloxamer onto drug crystals. Poloxamer, when used, is preferably present at a level between 1-20% by weight of the core; more preferably between 1-10% by weight of the core. A preferred poloxamer is Pluronic F127 (Poloxamer 407 available from BASF Corp.).

### Other Optional Excipients

The core formulation may optionally include one or more pharmaceutically acceptable excipients, carriers or diluents. Excipients are generally selected to provide good compression profiles during tablet compression. For example, a lubricant is typically used in a tablet formulation to prevent the tablet and punches from sticking in the die. Suitable lubricants include slippery solids such as talc, magnesium and calcium stearate, stearic acid, light anhydrous silicic acid, and hydrogenated vegetable oils. A preferred lubricant is magnesium stearate.

Other useful additives may include materials such as surface active agents (e.g., cetyl alcohol, glycerol monostearate, and sodium lauryl sulfate (SLS)), adsorptive carriers (e.g., kaolin and bentonite), preservatives, sweeteners, coloring agents, flavoring agents (e.g., citric acid, menthol, glycine or orange powder), stabilizers (e.g., citric acid, sodium citrate or acetic acid), dispersing agents, binders (e.g., hydroxypropylcellulose) and mixtures thereof. Typically such additives are present at levels below about 10% of the core weight; and for many such additives, they are typically present below about 1% of the core weight.

### Manufacturing Processes

The pharmaceutical core is prepared by methods that are well-known to those skilled in the art. For example, the components of the core (i.e., that portion of the tablet, exclusive of coatings, made on a tablet press) are generally mixed together, compressed into a solid form, the core is overcoated with a water-permeable coating, and then, if necessary, a delivery means through the water-permeable coating is provided (e.g., a hole is drilled in the coating to form an orifice). In some instances, the components can be simply mixed together and then compressed directly. However, it may be desirable for some formulations to be granulated by any technique known to those skilled in the art, followed by subsequent compression into a solid form.

The tablet core is generally prepared by standard tableting processes, such as by a rotary tablet press, which are well-known to those skilled in the art.

*Tablet Shape*

During the development of this dosage form, it was unexpectedly determined that the rate of drug delivery and the total amount of drug that remains within the dosage form after 24 hours in a dissolution medium (the residual) are greatly affected by the tablet shape. In particular, it was determined that the surface area for a given volume has an impact on the drug delivery. More specifically, it was determined that the surface area of the tablet after it swells in an aqueous environment for about one hour determines these factors. As such, although a standard SRC (standard radius concave) shape provides adequate drug delivery for useful delivery systems, as the mass to be delivered increases (and the corresponding tablet volume), it becomes increasingly advantageous to use shapes that provide a higher surface area to volume ratio. Measurements of volume and surface area can be accomplished by any standard method. For example, volume can be determined by liquid displacement. For example, a tablet swollen in a dissolution medium can be placed in a graduated cylinder containing water. The volume is determined by the change in the liquid meniscus before and after adding the tablet. Surface area can be estimated by caliper measurement (or using other non-contact measurement methods) of each axis, and using appropriate mathematics to calculate surface area. Alternatively, other surface area measurement techniques (e.g., BET measurements) can be used. As an example, for an SRC tablet, three parameters define the tablet dimensions: D for the diameter, cd for the cup depth, and t for thickness. The surface area can be calculated as

$$\text{Surface area} = 2\Pi[(D/2)(t-2cd) + (D/2)^2 + cd^2]$$

For the present invention, preferred surface area to volume ratios are greater than about 0.6 mm$^{-1}$; still more preferred are greater than 1.0 mm$^{-1}$. Three particular shapes are preferred: (1) an oblong shape, characterized by having no flat surfaces where twinning during coating can be an issue and a ratio of dimensions (for the die and punch) being about 1.3 to 3; more preferably between 1.5 and 2.5; (2) a caplet shape having a ratio of dimensions (for the die and punch) being about 1.3 to 3; more preferably between 1.5 and 2.5; and (3) a circular tablet where the faces of the tablet are opposed and inverted (i.e., curved inwardly) rather than curved outwardly as in a standard tablet. The most preferred shape is the oblong shape. Each of the preferred shapes is engineered to contain a single orifice or passageway.

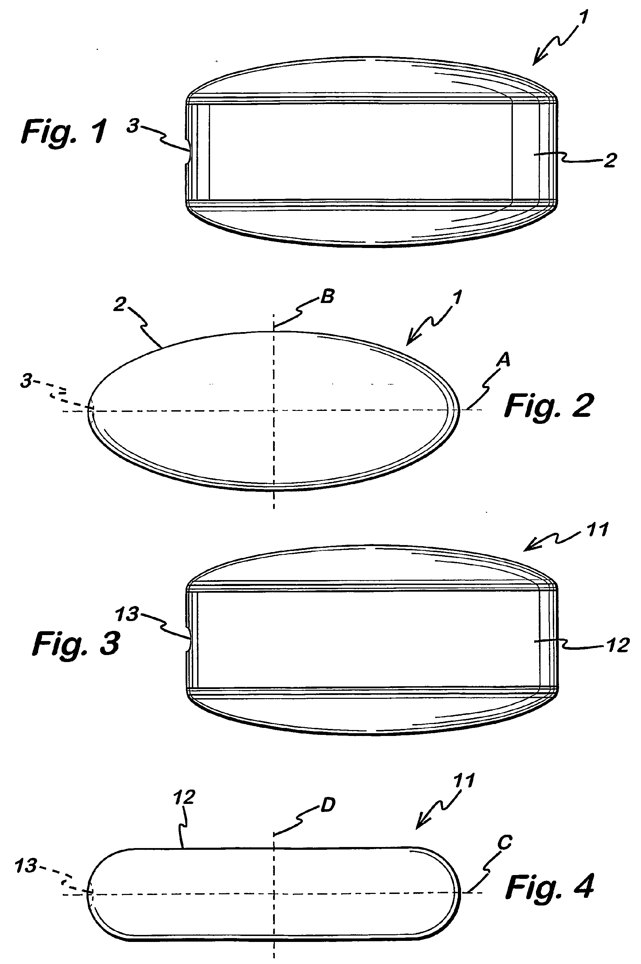

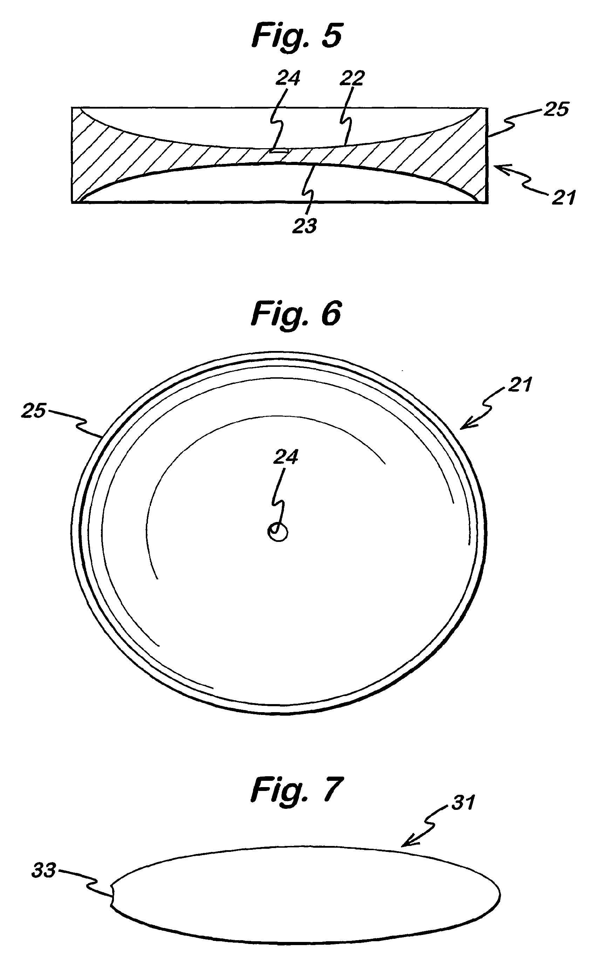

Preferred tablet shapes for use in the present invention are illustrated in the Figures, with Figures 1 and 2 representing a first preferred shape, Figures 3 and 4 representing a second preferred shape, and Figures 5 and 6 representing a third preferred shape.

With reference to Figures 1 and 2, tablet 1 is generally oblong in shape, and characterized by having no flat surfaces that disadvantageously promote twinning during coating. The tablet is further characterized by major axis A and minor axis B, as shown in Figure 2. Axes A and B determine the oblong or oval shape of the tablet (i.e., as seen from the top) and thus define tablet tooling axes in the tablet press. A preferred ratio of dimensions of major to minor axis (for the die and punch) is between 1.3 and 3; more preferably between 1.5 and 2.5. Tablet 1 can optionally further possess a band 2, normal to the oblong cross section shown in Figure 2. The midline of band 2 intersects both the major and minor axes, and the band surrounds and extends around the tablet, essentially encircling it. Tablet 1 is further characterized by an exit orifice 3, preferably the only orifice in the tablet: Orifice 3 is most preferably positioned as shown in Figure 1 such that it is ideally located at or near (within 3 mm) either of the two intersections of the (imaginary) midline (not shown) of band 2 with major axis A. Ideally, major axis A coincides with and/or crosses through the geometric midpoint of orifice 3. For example, if orifice 3 is a circular hole, major axis A ideally passes normal to and through its center. It is preferred that only a single orifice, located as just described, be used. The orifice preferably has a diameter between 500 and 1100 $\mu$m.

It is noted that the tablet of FIGURES 1 and 2 represents a preferred embodiment. An alternative embodiment is a tablet without the band shown in FIGURE 1, such that the side view is that of tablet 31 as shown in FIGURE 7, wherein the orifice is shown at 33.

With reference to Figures 3 and 4, tablet 11 is generally a caplet shape. Seen from the top as in Figure 4, it may be characterized as a cylinder portion terminating in hemispherical end portions. The tablet is otherwise analogous with that disclosed in Figures 1 and 2. Thus, the tablet is further characterized by major axis C and minor axis D, also shown in Figure 4. A preferred ratio of dimensions of major axis to minor axis (for the die and punch) is between 1.3 and 3; more preferably between 1.5 and 2.5. Tablet 11 further possesses a band 12 which extends around the tablet, analogous to that discussed above in Figures 1 and 2. Tablet 11 is further characterized by an exit orifice 13. Orifice 13 is positioned as shown in Figures 3 and 4 such that the it is ideally located at the intersection of the midline of band 12 with major axis C. Ideally, major axis C coincides with and/or crosses through the geometric midpoint of orifice 13. For example, if orifice 3 is a circular hole, major axis C ideally passes through its center.

With reference to Figures 5 and 6, tablet 21 is circular and two-sided, wherein the sides are opposing, as shown in the top view of Figure 6. Such a tablet shape is referred to as "lozenge shaped" in "Tableting Specification Manual, 5th Ed. (published by the American Pharmaceutical Association, Washington D.C. 2001). Although the top view is circular, the tablet is not spherical, but, as previously noted, has opposing faces 22 and 23, as shown in Figure 5 in cross section, each face being arched inwardly, rather than curved outwardly as in a standard tablet. Orifice 24 is preferably located in the geometric center of one of the circular, faces, as shown in the Figures. The surrounding edge 25 of the tablet can be thought of as analogous to the bands (2 and 12) of the other two shapes 1 and 11.

*Water-permeable Coating*

After compression, the tablet cores are ejected from the die. The cores are then overcoated with a water-permeable coating using standard procedures well-known to those skilled in the art. The water-permeable coating contains at least one passageway through which the drug is substantially delivered from the device. Preferably, the drug is delivered through the passageway as opposed to delivery primarily via permeation through the coating material itself. The term "passageway", "hole", and "orifice" are used interchangeably and refer to an opening or pore whether made mechanically, by laser drilling, *in situ* during use or by rupture during use. The passageway can extend into the core. However, since drilling a significant distance into the core can lead to loss of potency (and potential degradation if laser drilled), it is preferred that the penetration depth into the core be be less than 10% of the diameter of the tablet at that point, preferably less than 5%. Preferably, the passageway is provided by laser or mechanical drilling. The water-permeable coating can be applied by any conventional film coating process well known to those skilled in the art (e.g., spray coating in a pan or fluidized bed coating). The water-permeable coating is generally present in an amount ranging from about 3 wt% to about 30 wt%, preferably from about 6 wt% to about 15 wt%, relative to the core weight.

A preferred form of the coating is a water-permeable polymeric membrane. The passageway(s) may be formed either prior to or during use. The thickness of the polymeric membrane generally varies between about 20 $\mu$m and about 800 $\mu$m, and is preferably in the range of about 100 $\mu$m to about 500 $\mu$m. The size of the passageway will be determined by the particle size of the drug, the number of passageways in the device, and the desired delivery rate of the drug during operation. A typical passageway has a diameter from about 25 $\mu$m to about 2000 $\mu$m, preferably from about 300 $\mu$m to about 1200 $\mu$m, more preferably from about 400 $\mu$m to about 1000 $\mu$m. The passageway(s) may be formed post-coating by mechanical or laser drilling or may be formed *in situ* by rupture of the coatings. Rupture of the coating may be controlled by intentionally incorporating a relatively small weak portion into the coating. Passageways may also be formed *in situ* by erosion of a plug of water-soluble material or by rupture of a thinner portion of the coating over an indentation in the core. Multiple holes can be made in the coating. However, it is preferred to have oblong tablets with a single hole at one end of the tablet.

Coatings may be dense, microporous or "asymmetric," having a dense region supported by a thick porous region such as those disclosed in U.S. Patent Nos. 5,612,059 and 5,698,220. When the coating is dense, the coating is composed of a water-permeable material. When the coating is porous, it may be composed of either a water-permeable or a water-impermeable material.

Examples of osmotic devices that utilize dense coatings include U.S. Patent Nos. 3,995,631 and 3,845,770. The dense coatings are permeable to the external fluid such as water and may be composed of any of the materials mentioned in these patents as well as other water-permeable polymers known in the art.

The membranes may also be porous as disclosed in U.S. Patent Nos. 5,654,005 and 5,458,887 or even be formed from water-resistant polymers. U.S. Patent No. 5,120,548 describes another suitable process for forming coatings from a mixture of a water-insoluble polymer and a leachable water-soluble additive. The porous membranes may also be formed by the addition of pore-formers as disclosed in U.S. Patent No. 4,612,008.

In addition, vapor-permeable coatings may even be formed from extremely hydrophobic materials such as polyethylene or polyvinylidenefluoride that, when dense, are essentially water-impermeable, so long as such coatings are porous.

Materials useful in forming the coating include various grades of acrylics, vinyls, ethers, polyamides, polyesters and cellulosic derivatives that are water-permeable and water-insoluble at physiologically relevant pH's, or are susceptible to being rendered water-insoluble by chemical alteration such as by crosslinking.

Specific examples of suitable polymers (or crosslinked versions) useful in forming the coating include plasticized, unplasticized and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CA ethyl carbonate, CA chloroacetate, CA ethyl oxalate, CA methyl sulfonate, CA butyl sulfonate, CA p-toluene sulfonate, agar acetate, amylose triacetate, beta-glucan acetate, beta glucan triacetate, acetaldehyde dimethyl acetate, triacetate of locust bean gum, hydroxlated ethylene-vinylacetate, ethyl cellulose (EC), polyethylene glycol (PEG), polypropylene glycol (PPG), PEG/PPG copolymers, polyvinylpyrrolidone (PVP), hydroxyethyl cellulose (HEC), hydroxypropyl cellulose (HPC), carboxymethyl cellulose (CMC), carboxymethylethyl cellulose (CMEC), hydroxypropylmethyl cellulose (HPMC), hydroxypropylmethyl cellulose propionate (HPMCP), hydroxypropyl methylcellulose acetate succinate (HPMCAS), poly(acrylic) acids and esters and poly(methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes and synthetic waxes.

A preferred coating composition comprises a cellulosic polymer, in particular cellulose ethers, cellulose esters and cellulose ester-ethers, i.e., cellulosic derivatives having a mixture of ester and ether substituents, such as HPMCP.

Another preferred class of coating materials are poly(acrylic) acids and esters, poly(methacrylic) acids and esters, and copolymers thereof.

A more preferred coating composition comprises cellulose acetate. Preferred cellulose acetates are those with acetyl contents between 35% and 45% and number-average, molecular weights ($MW_n$) between 30,000 and 70,000. An even more preferred coating comprises a cellulosic polymer and PEG. A most preferred coating comprises cellulose acetate and PEG. A preferred PEG has a weight-average molecular weight from about 2000 to about 5000; more preferred between 3000 and 4000.

The coating process is conducted in conventional fashion, typically by dissolving the coating material in a solvent and then coating by dipping, fluid bed coating, spray-coating or preferably by pan-coating. A preferred coating solution contains 5 to 15 weight percent polymer. Typical solvents useful with the cellulosic polymers mentioned above include acetone, methyl acetate, ethyl acetate, isopropyl acetate, n-butyl acetate, methyl isobutyl ketone, methyl propyl ketone, ethylene glycol monoethyl ether, ethylene glycol monoethyl acetate, methylene dichloride, ethylene dichloride, propylene dichloride, nitroethane, nitropropane, tetrachloroethane, 1,4-dioxane, tetrahydrofuran, diglyme, and mixtures thereof. The use of water based latex or pseudo-latex dispersions are also possible for the coating. Such coatings are preferred due to the manufacturing advantages of avoiding organic solvents and potential environmental challenges therein. Pore-formers and non-solvents (such as water, glycerol and ethanol) or plasticizers (such as diethyl phthalate and triacetin) may also be added in any amount as long as the polymer remains soluble at the spray temperature. Pore-formers and their use in fabricating coatings are described in U.S. Patent No. 5,612,059. In general, more water-soluble additives (such as PEG) increase the water-permeability of the coating (and thereby the drug delivery rate) while water insoluble additives (such as triacetin) decrease the rate of drug delivery.

*Orifices*

During the development of this dosage form, it has been determined that the position and number of orifices (e.g., holes) can have a significant impact on the drug delivery rate and residual amount of drug remaining after 24 hours in a dissolution medium. In particular, Applicants have determined that a single hole drilled on the band of the tablet provides superior performance. For oblong or caplet-shaped tablets, the hole is preferably made on the band at one tip of the tablet (i.e., coincident with the major axis, as illustrated in Figure 1). The superior performance of such a hole position is shown in Example 11. The advantage of a hole on the end for oblong or caplet-shaped tablets is believed to be due to the ability of the shape to focus the final percentage of extrudable material to the exit hole.

*Additional Coatings*

It is often desirable to provide an additional coating or coatings on the inside or outside of the water-permeable coating. Coatings underneath the water-permeable coating are preferably permeable to water. Such coatings can serve to improve adhesion of the water-permeable coating to the tablet core, or to provide a chemical and/or act as a physical barrier between the core and the water-permeable coating. A barrier coating can insulate the core during coating to the water-permeable coating from, for example, the coating solvent or from migration of a plasticizer (e.g., PEG) during storage. External coatings can be cosmetic to help with product identification and marketing, and improve mouth feel and swallowability. Such coatings can also be functional. Examples of such functional coatings include enteric coatings (i.e., coatings designed to dissolve in certain regions in the gastrointestinal tract) and opacifying coatings (designed to block light from reaching a light-sensitive drug). Other product identifying features can also be added to the top of the coating. Examples include, but are not limited to, printing and embossing of identifying information. The additional coating can also contain an active pharmaceutical ingredient, either the same or different from that in the core. This can provide for combination drug delivery and/or allow for specific pharmacokinetics (e.g., pulsatile). Such a coating can be film coated with an appropriate binder onto the tablet core.

In addition, active material can be compression coated onto the tablet surface. In many cases, this compression coating can be facilitated by use of a compressible film coat as disclosed in co-pending U.S. Provisional Patent Application No. 60/275889 filed March 14, 2001.

*Packaging*

The osmotic tablets may be packaged in a variety of ways. Generally, an article for distribution includes a container which holds the osmotic tablets. Suitable containers are well-known to those skilled in the art and include materials such as bottles (plastic and glass), plastic bags, foil packs, and the like. The container may also include a tamper-proof assemblage to prevent indiscreet access to the contents of the package and a means for removing moisture and/or oxygen (e.g., oxygen absorbers such as D Series FreshPax™ packets available from Multisorb Technologies Inc., Buffalo, NY, USA, or Ageless™ and ZPTJ™ sachets available from Mitsubishi Gas Corporation, Tokyo, JP). The container typically has deposited thereon a label that describes the contents of the container and any appropriate warnings.

The following Examples illustrate the osmotic systems of the present invention. To exemplify the general concepts of the present invention, specific pharmaceutically active ingredients are used. However, those skilled in the art will appreciate that the particular drugs used are not limiting to the scope of the invention and should not be so construed.

EXAMPLES

Unless specified otherwise, starting materials are generally available from commercial sources such as Aldrich Chemicals Co. (Milwaukee, WI), Lancaster Synthesis, Inc. (Windham, NH), Acros Organics (Fairlawn, NJ), Maybridge Chemical Company, Ltd. (Cornwall, England), Tyger Scientific (Princeton, NJ), and AstraZeneca Pharmaceuticals (London, England) or can be made using standard procedures well-known to those skilled in the art. The following materials used in the Examples may be obtained from the corresponding sources listed below:

| | |
|---|---|
| Natrosol™ 250H and 250HX (hydroxyethylcellulose); and Klucel™ EXF, EF and HF (hydroxypropylcellulose) | Hercules Corporation, Aqualon Division, Wilmington, DE |
| Neosorb™ P110 and 30/60 DC (sorbitol); Pearlitol™ 100SD (mannitol); and Xylisorb™ 90 (xylitol) | Rouquette America, Inc. Keokuk, IA |
| Pharmacoat™ 603 (hydroxypropylmethylcellulose) | Shin-Etsu Chemical Corp., Tokyo, Japan |
| Sodium lauryl sulfate | Sigma-Aldrich St. Louis, MO |
| Magnesium stearate | Mallinckrodt Inc. Hazelwood, MO |
| Cellulose acetate (398-10) 39.8% acetyl content; 10 s falling ball viscosity | Eastman Chemicals, Kingsport, TN |
| Polyethylene glycol (PEG) 3350 | Union Carbide Corp. (subsidiary of Dow Chemical Co., Midland, MI) |
| Sucrose, extrafine granular | Tate & Lyle London, UK |

(continued)

| | |
|---|---|
| Sodium Chloride | Mallinckrodt Baker Inc. Phillipsburg, NJ |
| Ascorbic acid, medium 200 μm | Merck KGaA, Germany |
| Trehalose | Sigma-Aldrich Co. St. Louis, MO |
| Carbopol™ 974 PNF (poly(acrylic acid)) | Noveon Inc. Cleveland, OH |
| Xylitab™ 200 (xylitol) | Danisco Ingredients USA, Inc New Century, KS |
| Mannitol, powdered | EM Industries Inc. Hawthorne, NY |

Sertraline hydrochloride ((1S-cis)-4-(3,4-dichlorophenyl)-1,2,3,4-tetrahydro-N-methyl-1-naphthalenamine hydrochloride) was prepared using the general procedures described in U.S. Patent Nos. 4,536,518 and 5,248,699.

Sildenafil citrate (1-[[3-(6,7-dihydro-1-methyl-7-oxo-3-propyl-1H-pyrazolo[4,3-d]pyrimidin-5-yl)-4-ethoxyphenyl]sulfonyl]-4-methylpiperazine citrate) was prepared using the general procedures described in U.S. Patent No. 5,250,534.

[2-(3,4-Dichlorophenoxy)-5-fluorobenzyl]-methylamine hydrochloride, referred to as "Compound A" in the Examples, was prepared using the general procedures described in PCT Publication No. WO 0050380 (Example 56).

Unless specified otherwise, tablet cores were prepared using a Manesty™ F-Press (single-punch tablet machine available from Manesty Corporation, Liverpool, UK). Use of such tablet presses is described in Pharmaceutical Dosage Forms: Tablets, Volume 2 (H. A. Leberman, L. Lachman, J. B. Schwartz, Eds.), Marcel Dekker, Inc. New York (1990).

Example 1 illustrates the range of concentrations that may be used of a high molecular weight hydroxyethylcellulose (HEC) in an osmotic core formulation and still deliver at least about 80% of the drug.

<u>Example 1</u>

Blends were prepared by mixing each of six test blends indicated in Table I below in a Turbula™ blender (available from Glen Mills Inc., Clifton, NJ) for 20 minutes (without the magnesium stearate).

**Table I**

| Ingredient | 1-1 | 1-2 | 1-3 | 1-4 | 1-5 | 1-6 |
|---|---|---|---|---|---|---|
| Sertraline HCl | 30.0 g | 30.0 g | 30.0 g | 30.0 g | 30.0 g | 30.0 g |
| Klucel™ EXF | 5.0 g | 5.0 g | 5.0 g | 5.0 g | 5.0 g | 5.0 g |
| Sodium laurel sulfate | 0.7 g | 0.7 g | 0.7 g | 0.7 g | 0.7 g | 0.7 g |
| Natrasol™ 250H | 0.0 g | 3.0 g | 6.0 g | 10.0g | 15.0g | 30.0 g |
| NeosorbTM 30/60 DC | 63.3 g | 60.3 g | 57.3 g | 53.3 g | 48.3 g | 33.3 g |
| Magnesium stearate | 1.0 g | 1.0 g | 1.0 g | 1.0 g | 1.0 g | 1.0 g |

The magnesium stearate was then added to each sample followed by an additional 5 minutes of mixing. Tablet cores (i.e., uncoated) were prepared on a Manesty F-press using 5/16" (0.79 cm) SRC punch and die set with tablet core weight averages of 299 mg. Tablet cores were then coated with a solution of cellulose acetate, polyethylene glycol 3350, acetone and water with a weight ratio of 4.1/1.9/89.0/5.0. Coatings were carried out using a Vector Hi-Coater LDCS 20 (available from Vector Corporation, Marion, IA) to give a total tablet weight of 328 mg. Each tablet was mechanically drilled with a 0.6 mm drill bit to give one hole through the coating.

Dissolution experiments were carried out in a Hanson SR8 dissolution apparatus (available from Hanson Research Corp., Chatsworth, CA) using USP2 paddles (37°C; 50 RPM). The dissolution medium (900 mL per sample) was an acetate buffer at pH 4.5. Following dissolution, samples were analyzed using a TEA/Phosphate buffer (pH 6.6) with THF (38%) and methanol (16%). Samples were analyzed using an HP1100 series HPLC with a Waters Symmetry C-18, 5-$\mu$m column (45°C; 1 mL/min flow; 20 $\mu$L injection volume; 275 nm detection). The results from this study are shown in Table II below (reported as percent dissolved in the dissolution medium as a function of time):

**Table II**

| Test Sample | 6 hrs | 8 hrs | 12 hrs | 24 hrs |
|---|---|---|---|---|
| **1-1**<br>(0% HEC) | 7% | 13% | 23% | 44% |
| **1-2**<br>(3% HEC) | 42% | 55% | 69% | 84% |
| **1-3**<br>(6% HEC) | 53% | 62% | 71% | 86% |
| **1-4**<br>(10% HEC) | 37% | 54% | 77% | 88% |
| **1-5**<br>(15% HEC) | 40% | 55% | 72% | 87% |
| **1-6**<br>(30% HEC) | 46% | 62% | 76% | 89% |

Example 2 compares the use of a high molecular weight hydroxyethylcellulose to other commonly used water-soluble polymers in an osmotic core formulation.

Example 2

Tests of the effect of different polymers on drug delivery were investigated by preparing tablets by a common procedure using the formulations outlined in Table III below.

**Table III**

| Component | 2-1 | 2-2 | 2-3 | 2-4 |
|---|---|---|---|---|
| Sildenafil citrate | 24.30 g | 24.30 g | 24.30 g | 24.30 g |
| Xylisorb™ 90 | 18.42 g | 18.42 g | 0.0 g | 18.42 g |
| Mannitol Pearlitol™ 100SD | 0.0 g | 0.0 g | 18.42 g | 0.0 g |
| Carbopol™ 974 PNF | 6.86 g | 0.0 g | 0.0 g | 0.0 g |
| HPC Klucel™ HF | 0.0 g | 6.86 g | 0.0 g | 0.0 g |
| HPMC Pharmacoat™ 603 | 0.0 g | 0.0 g | 6.86 g | 0.0 g |
| HEC Natrasol™ HX | 0.0 g | 0.0 g | 0.0 g | 6.86 g |

Blends were prepared by first Turbula™ mixing the above components for 15 minutes. The mixtures were then screened through a 250 $\mu$m mesh sieve and blended again for an additional 15 minutes. To each of the above mixtures was added 0.43 g of magnesium stearate, and the blends were Turbula mixed for an additional 5 minutes. Tablet cores were prepared using an F-press with 7/16" (1.1 cm) SRC tooling to give 587 mg/tablet (equivalent to 200 mg of sildenafil free base per tablet). A coating fluid was prepared by dissolving 35 g of cellulose acetate and 15 g of PEG 3350 in 925 g of acetone and 25 g of water. Tablet cores were coated on an LDCS 20 coater (available from Vector Corp.) to give a weight gain of between 6 and 8%. One hole was mechanically drilled in each tablet using a 500 $\mu$m drill bit. The results of the dissolution experiments (at pH 2) are shown below in Table IV (reported as percent dissolved in the dissolution medium as a function of time). The pH 2 medium was a buffer 0.089 N NaCl/0.01 N HCl. Dissolution experiments were carried out in 900 mL of solution per tablet using a CSP Vankel™ dissolution apparatus using baskets at 100 rpm and a temperature of 37°C. Analysis was conducted by ultraviolet absorption at 292 nm.

**Table IV**

| Test Sample | 8 hours | 12 hours | 16 hours | 24 hours |
|---|---|---|---|---|
| **2-1** (Carbapol) | 18 | 19 | 20 | 23 |
| **2-2** (HPC) | 0 | 0 | 3 | 13 |
| **2-3** (HPMC) | 4 | 8 | 30 | 53 |
| **2-4** (HEC) | 67 | 76 | 79 | 84 |

Example 3 further illustrates the difference between HEC and other polymers (polyethylene oxides) commonly used in osmotic systems.

Example 3

A blend was prepared using 125 g of sertraline hydrochloride, 242.5 g of sorbitol (Neosorb 30/60 DC), 3.5 g of sodium lauryl suflate and 25 g of Klucel EXF by Turbula blending in a bottle for 30 mins. To sample 1, 1 g of Natrasol 250H HEC, 3 g of Neosorb 30/60 DC and 15.8 g of the above blend were added. To sample 2, 1 g of Polyox WSR coagulant grade (Union Carbide Corp.), 3 g of Neosorb 30/60 DC and 15.8 g of the above blend were added. To sample 3, 1 g of Polyox WSR N80 (Union Carbide Corp.), 3 g of Neosorb 30/60 DC and 15.8 g of the above blend were added. Each sample was Turbula blended for 10 minutes. To each of three bottles, 0.2 g of magnesium stearate were added followed by an addition 5 minutes of Turbula blending. Tablet cores of each sample were prepared using an F-press with 5/16" SRC tooling to give tablets of 300 mg (hardnesses from 10-12 kP). Tablets were then coated with a solution of cellulose acetate/polyethylene glycol 3350/acetone/water in a weight ratio of 4.1/1.9/89.0/5.0. Coatings were carried out using a Vector Hi-Coater LDCS 20 (available from Vector Corporation, Marion, IA) to give a total tablet weight corresponding to a 6% weight gain. Each tablet was mechanically drilled with a 0.6 mm drill bit to give one hole through the coating. Dissolution was carried out using USP Type II analysis with paddles at 50 rpm, 900 mL of 50 mM sodium acetate buffer (pH 4.5) at 37°C. Analysis was carried out as described in Example 1. The results are shown in Table V expressed as percent dissolved as a function of time.

**Table V**

| Test Sample | 8 hours | 12 hours | 24 hours |
|---|---|---|---|
| **3-1** (HEC) | 60 | 70 | 83 |
| **3-2** (Polyox Coag.) | 42 | 59 | 71 |
| **2-3** (Polyox N80) | 10 | 30 | 72 |

Example 4 illustrates the importance of high molecular weight HEC vs. low molecular weight HEC.

Example 4

Tests of the effect of different formulations on drug delivery were investigated by preparing tablets by a common procedure using the formulations outlined in Table V below.

**Table VI**

| Component | 4-1 | 4-2 | 4-3 |
|---|---|---|---|
| Sertraline HCl | 89.59 g | 89.59 g | 89.59 g |
| mannitol 2080 | 24.79 g | 27.11 g | 29.26 g |
| Dextrose | 24.79 g | 26.94 g | 29.26 g |
| HPMC 2910 | 10.58 g | 10.58 g | 10.58 g |
| Sodium lauryl sulfate | 1.16 g | 1.16 g | 1.16 g |
| HEC 250H | 8.93 g | 8.93 g | 0.0 g |
| HEC 250L | 4.46 g | 0.0 g | 4.46 g |

Blends were prepared by mixing each of three test blends indicated in Table VI above in a Turbula™ blender (available from Glen Mills Inc., Clifton, NJ) for 20 minutes. To each of the test blends was added 0.83 g of magnesium stearate and then each was Turbula mixed for an additional 5 minutes. Tablet cores were prepared using an F-press with 1/4" (6.35 mm) SRC tooling to give cores with an average weight of 165.3 mg (corresponding to 80 mgA). Cores were then coated with a solution of cellulose acetate/polyethylene glycol 3350/acetone/water in a weight ratio of 4.1/1.9/89.0/5.0. Coatings were carried out using a Vector Hi-Coater LDCS 20 (available from Vector Corporation, Marion, IA) to give a total tablet weight corresponding to a 6% weight gain. Each tablet was mechanically drilled with a 0.6 mm drill bit to give one hole through the coating. Analysis was carried out as described in Example 1. The results are shown in Table VII expressed as percent dissolved as a function of time.

**Table VII**

| Test Sample | 6 hrs | 8 hrs | 12 hrs | 22 hrs |
|---|---|---|---|---|
| **4-1** | 70 | 77 | 80 | 93 |
| **4-2** (no HEC-L) | 58 | 70 | 79 | 92 |
| **4-3** (no HEC-H) | 21 | 26 | 39 | 72 |

Example 5 illustrates the use of the invention with an antidepressive drug and the performance of the tablets *in vivo* (dogs).

Example 5

An 80 mg sertraline HCl tablet (based on the free base) was prepared using the following procedure: A Niro SP1 high shear granulator with a 10 L bowl was charged with 600 g of sertraline hydrochloride, 200 g of Natrosol™ 250H, 100 g of Klucel™ EXF, 14 g of sodium laurel sulfate and 1146 g of sorbitol (Neosorb™ P110). A mixture of 9:1 isopropyl alcohol:water (460 g) was added under high shear to form the granulation. The granulation was tray-dried in an oven at 40°C for 16 hours. The granulation was then milled using an M5A mill equipped with a 0.030 inch (0.762 mm) Conidur rasping screen at 300 rpm. The material was combined with 20 g of magnesium stearate and blended in a V-blender for five minutes. The powder was tableted using a Kilian™ T100 tablet press (available from Kilian & Co., Inc., IMA Solid Dose Division, Horsham, PA) with 5/16" (8 mm) SRC tooling to give tablet cores weighing 299 mg. The tablets were coated using a Vector HCT-30 EP HiCoater (available from Vector Corporation, Marion, IA). The coating solution was 4.1/1.9/89.0/5.0 (weight ratios) of cellulose acetate/PEG 3350/acetone/water. The coating was carried out until a weight gain of 10% of the tablet core initial weight was achieved. The resulting tablets were dried for 16 hours at 40°C in an oven. One 900-$\mu$m hole was drilled in each tablet face using a mechanical drill. *In vitro* dissolution shows that about 50% of the drug is delivered in 6 hours and 90% in 24 hours, at pH 4.5. Three dogs were dosed four tablets at a time. The process was then repeated after several days. Tablets were recovered from the feces and analyzed for residual drug remaining in the core. The residence time in the dogs was recorded based on approximate defecation times. Results were as follows: 9-17 hours, 67.6$\pm$ 0.6% delivered (sample size = 8); 20-27 hours, 68.5$\pm$0.6% delivered (sample size = 12); 30-40 hours, 82$\pm$1% delivered (sample size = 4). As a comparison, the *in vitro* dissolution behavior at pH 6.8 (simulated intestinal fluid) yields approximately 62% for 9-17 hours, 72% for 20-27 hours and 85% for 30-40 hours.

Example 6 illustrates the flexibility of use of different osmagents while maintaining good drug delivery performance.

Example 6

Test materials were prepared by combining the materials listed in Table VIII and tested using the procedures described in Example 2.

**Table VIII**

| Ingredient | 6-1 | 6-2 | 6-3 | 6-4 | 6-5 |
|---|---|---|---|---|---|
| Sildenafil citrate | 24.3 g | 24.3 g | 24.3 g | 24.3 g | 24.3 g |
| Natrasol™ 250 HX | 3.43 g | 3.43 g | 3.43 g | 3.43 g | 3.43 g |
| Pharmacoat™ 603 | 3.43 g | 3.43 g | 3.43 g | 3.43 g | 3.43 g |
| Pearlitol™ (mannitol) | 18.42 g | 0.0 g | 0.0 g | 0.0 g | 0.0 g |
| Neosorb™ P100T (sorbitol) | 0.0 g | 18.42 g | 0.0 g | 0.0 g | 0.0 g |
| Xylisorb™ 90 (xylitol) | 0.0 g | 0.0 g | 18.42 g | 0.0 g | 0.0 g |
| Trehalose | 0.0 g | 0.0 g | 0.0 g | 18.42 g | 0.0 g |
| Sodium chloride | 0.0 g | 0.0 g | 0.0 g | 0.0 g | 18.42 g |

To each bottle was added 0.43 g of magnesium stearate, and the mixtures were Turbula blended an additional 5 minutes. Tablet cores were prepared using an F-press with 7/16" (1.1 cm) SRC tooling to give 587 mg/tablet (equivalent to 200 mg of sildenafil free base per tablet). A coating fluid was prepared by dissolving 35 g of cellulose acetate and 15 g of PEG 3350 in 925 g of acetone and 25 g of water. Tablets were coated on an LDCS20 coater to give a weight gain of between 6 and 8%. One hole was mechanically drilled in each tablet using a 500 μm drill bit. The results of the dissolution experiments (at pH 2) are shown below in Table IX (reported as percent dissolved in the dissolution medium as a function of time):

**Table IX**

| Sample | 8 hrs | 12 hrs | 16 hrs | 24 hrs |
|---|---|---|---|---|
| **6-1** (mannitol) | 45% | 64% | 76% | 82% |
| **6-2** (sorbitol) | 48% | 63% | 74% | 87% |
| **6-3** (xylitol) | 57% | 71% | 78% | 88% |
| **6-4** (trehalose) | 39% | 56% | 66% | 79% |
| **6-5** (NaCl) | 48% | 63% | 69% | 76% |

Example 7 illustrates the use of a soluble acid as both a bioavailability enhancing excipient (based on its expected behavior *in vivo)* and as an osmagent.

Example 7

A blend was prepared by Turbula-mixing the following components for 20 minutes: 24.3 g of sildenafil citrate, 2.14 g of Natrasol™ 250HX, 2.14 g of Pharmacoat™ 603, 4.29 g of Xylisorb™ 90 and 16.71 g of ascorbic acid. To this mixture was added 0.43 g of magnesium stearate followed by an additional 5 minutes of Turbula-mixing. Tablet cores were prepared using an F-press with 7/16" (1.1 cm) SRC tooling to give a 587 mg/tablet (equivalent to 200 mg of sildenafil free base per tablet). A coating fluid was prepared by dissolving 35 g of cellulose acetate and 15 g of PEG 3350 in 925 g of acetone and 25 g of water. Tablets were coated on an LDCS20 coater to give a weight gain of between 6 and 8%. One hole was mechanically drilled in each tablet using a 500 $\mu$m drill bit. The results of the dissolution experiments (at pH 2) show that the release at 8, 12, 16 and 24 hours correspond to 68%, 77%, 81% and 87%, respectively.

Example 8 further illustrates the versatility of the invention.

Example 8

A tablet containing [2-(3,4-dichlorophenoxy)-5-fluorobenzyl]-methylamine hydrochloride (300 mg based on the free base) was prepared using the following procedure. A high shear granulating unit (Niro SP1, 10 L bowl) was charged with 1015.2 g of drug, 375.7 g of sucrose (extra fine granular), 375.7 g of mannitol powder, 121.8 g of hydroxyethylcellulose (Natrosol™ 250HX), 20.3 g of sodium lauryl sulfate, and 101.7 g of hydroxypropylcellulose (Klucel™ EF). The components were dry mixed for five minutes at an impeller speed of 300 rpm and a chopper speed of 1000 rpm. A mixture of 9:1 isopropyl alcohol:water (410 mL) was added within 5 minutes under high shear (impeller speed of 500 rpm, chopper speed of 1000 rpm). The sample was mixed under high shear an additional minute following the liquid addition. The granulation was tray-dried in an oven at 40°C for 16 hours. The granulation was then milled using a Fitzpatrick M5A mill equipped with a 0.030" (0.762 mm) Conidur rasping screen using a bar rotor at 300 rpm. The material was then combined with 19.6 g of magnesium stearate and blended in an 8-quart V-blender for five minutes. The powder was tableted using a Kilian rotary tablet press with 7/16" (1.1 cm) SRC tooling to give tablet cores weighing 675 mg (300 mg of drug based on the free base). The resulting tablet cores were coated using an LDCS-20 Hi-coater. The coating solution was 4.1/1.9/89/5.0 (weight ratios) of cellulose acetate/PEG 3350/acetone/water. The coating was carried out until a weight gain of 6-8% of the tablet core initial weight was achieved. The resulting tablets were dried for 16 hours at 40°C in an oven. One 900-$\mu$m hole was drilled in one tablet face using a mechanical drill. *In vitro* dissolution at pH 4.5 (sodium acetate buffer) shows that 84% of the drug is delivered in 12 hours and 97% in 24 hours.

Example 9 illustrates the incorporation and beneficial effect of addition of an acid to a formulation.

Example 9

Tablets of [2-(3,4-dichlorophenoxy)-5-fluorobenzyl]-methylamine hydrochloride (300 mg based on the free base) were prepared by combining 15.2 g of drug, 5.6 g of sorbitol, 5.6 g of tartaric acid, 1.8 g of hydroxyethyl cellulose (Natrosol 250 HX), 0.3 g of sodium lauryl sulfate, and 1.5 g of hydroxypropyl cellulose (Klucel-EF). The mixture was blended in an amber glass bottle using a Turbula mixer for 20 minutes. The blend was charged into a mini-granulating unit and a mixture of 9:1 isopropyl alcohol:water (7-8 mL) was added within 6.5 minutes under high shear. The granulation was tray-dried in an oven at 40°C for 16 hours. The granulation was then milled using a Fitzpatrick L1A mill equipped with a 0.030" (0.762 mm) Conidur rasping screen at 300 rpm. The material was combined with 0.3 g of magnesium stearate and bottle blended using a Turbula mixer for five minutes. The powder was tableted using the Manesty F-Press with 7/16" (1.1 cm) SRC tooling to give tablet cores weighing 675 mg. The tablets were coated using an LDCS-20 coater. The coating solution was 4.1/1.9/89/5.0 (by weight) of cellulose acetate (398-10; Eastman Chemicals)/PEG 3350/acetone/water. The coating was carried out until a weight gain of 6-8% of the tablet core initial weight was achieved. The resulting tablets were dried for 16 hours at 40°C in an oven. One 900 $\mu$m hole was drilled in one tablet face using a mechanical drill. *In vitro* dissolution in pH 7.5 simulated intestinal fluid (potassium phosphate buffer) shows that 61% of the drug is delivered in 12 hours and 68% of the drug is delivered in 16 hours in formulations containing tartaric acid. In contrast, formulations prepared as in Example 9 (without tartaric acid) delivered 52% in 12 hours and 53% in 16 hours.

Example 10 shows the benefit of the caplet shape compared to the standard SRC shape for the tablet.

Example 10

A blend of 49.5 grams of Neosorb P110, 50 grams of [2-(3,4-dichlorophenoxy)-5-fluorobenzyl]-methylamine hydrochloride and 0.25 grams of magnesium stearate was bottle blended, sieved, and then turbula mixed. A Fitzpatrick IR220 Chilsonator was used to roller compact the blend. The resulting ribbons were milled with a mortar and pestle. 97.8 grams of the milled material was combined with 11.6 grams of Natrosol 250H NF and 5.8 grams of Klucel EXF, bottle blended, sieved and turbula mixed. 1.2 grams of magnesium stearate was added and turbula mixed for 4 min. Tablet cores were prepared by first roller compacting together a 1:1 (wt:wt) mixture of Compound A and sorbitol. A blend was prepared by combining 84% of the above mixture with 10% Natrosol 250H NF and 5% Klucel EXF in a bottle. The material was turbula blended for 20 minutes. At this point, 1% magnesium stearate was added and the blend was turbula mixed for an additional 5 minutes. Tablet cores were prepared using an F-press with either 7/16" SRC or 0.254 X 0.748" caplet tooling to give 629 mg/tablets. Cores were coated with a solution of 4% (w:w) cellulose acetate 398-10, 2% PEG 3350, 89% acetone and 5% water using a Vector LDCS20 Hicoater to a weight gain of 9-10% of the mean tablet core weight. Single holes (0.9 mm) were mechanically drilled either on the face of the SRC tablet or the end of the caplet in the band. Dissolution was performed using medium consisting of 50 mM sodium acetate at pH 4.5 at 37°C using USP Apparatus 2 at 50 rpm. An HPLC was used to analyze drug concentrations in solution. The time to deliver 50% of the drug was 10.4±2.2 hrs for the SRC shaped tablet and 6.3±0.3 hrs for the caplet shape.

Example 11 shows the benefit of positioning the hole for a caplet shape.

Example 11

A blend of 49.5 grams of Neosorb P110, 50 grams of [2-(3,4-dichlorophenoxy)-5-fluorobenzyl]-methylamine hydrochloride and 0.25 grams of magnesium stearate was bottle blended, sieved, and then Turbula mixed. A Fitzpatrick IR220 Chilsonator was used to roller compact the blend. The resulting ribbons were ground with a mortar and pestle. 97.8 grams of the ground material was combined with 11.6 grams of Natrosol 250H NF, 5.8 grams of Klucel EXF, bottle blended, sieved and turbula mixed. 1.2 grams of magnesium stearate was added and turbula mixed for 4 min. Tablet cores were prepared by first roller compacting together a 1:1 (wt:wt) mixture of Compound A and sorbitol. A blend was prepared by combining 84% of the above mixture with 10% Natrasol 250H and 5% Klucel EXF in a bottle. The material was turbula blended for 20 minutes. At this point, 1% magnesium stearate was added and the blend was turbula mixed for an additional 5 minutes. Cores were prepared using an F-press with 0.254 X 0.748" caplet tooling to give 629 mg/ tablet core. Cores were coated with a solution of 4% (w:w) cellulose acetate 398-10, 2% PEG 3350, 89% acetone and 5% water using a Vector HCT20 LDCS20 Hicoater to a weight gain of 9-10% of the mean tablet core weight. Holes (0.9 mm) were mechanically drilled either (1) on the face of the tablet; (2) 1 hole on the end in the band; or (3) 1 hole on each end in the band. Dissolution was performed in 50 mM sodium acetate at pH 4.5 at 37°C using USP Apparatus 2 at 50 rpm. HPLC was used to analyze drug concentrations in solution. Dissolution was performed using a medium consisting of 50 mM sodium acetate at pH 4.5 at 37°C using an HPLC potency analysis. The time to deliver 50% of the drug was 8.2±0.2 hrs for one hole in the tablet face; 6.3±0.3 hrs for one hole on the end of the tablet; and 6.8±0.3 hrs for one hole on each end of the tablet.

Example 12 shows the benefit of a lozenge-shaped tablet.

Example 12

Tablet cores were prepared by combining 42% (w:w) [2-(3,4-dichlorophenoxy)-5-fluorobenzyl]-methylamine hydrochloride, 42% Neosorb 30/60DC, 10% Natrosol 250H and 5% Klucel EXF, hand sieving through a #20 sieve, then blending in a bottle using a turbula blender for 20 minutes. Magnesium stearate (1%) was then added followed by an additional 5 minutes of blending. Cores were prepared using a 7/16" SRC (control) to give 629 mg/tablet, and using a 7/16" flat faced beveled edge giving approximately 750 mg/tablet. The flat-faced cores were then placed on a lathe and carved to the desired shape, stopping as the cores reached 629 mg/core. Cores were coated with a solution of 4% (w: w) cellulose acetate 398-10, 2% PEG 3350, 89% acetone and 5% water using a Vector HCT20 LDCS20 Hicoater to a weight gain of 9-10% of the mean tablet core weight. Single holes (0.9 mm) were mechanically drilled on the face of the tablets. Dissolution was performed in 50 mM sodium acetate at pH 4.5 at 37°C using USP Apparatus 2 at 50 rpm. HPLC was used to analyze drug concentrations in solution. Dissolution was performed using a medium consisting of 50 mM sodium acetate at pH 4.5 at 37°C using an HPLC potency analysis. The time to deliver 50% of the drug was 9.4 hrs for the SRC control and 6.57.6 hrs for the indented SRC shape.

Example 13 illustrates the advantage of the addition of a dispersant to the dosage form.

Example 13

Dispersant-coated drug was prepared by first dissolving 1.00 g of Pluronic F127 NF in 15.0 g of ethanol. The drug [2-(3,4-dichlorophenoxy)-5-fluorobenzyl]-methylamine hydrochloride (10.09 g) was then slurried with the ethanol solution. The material was dried in an oven for 12 hours at 50°C. Material was sieved through a #40 screen. A test blend was prepared by combining 7.20 g of the above material with 0.75 g of Klucel EXF, 0.90 g of HEC Natrosol 250 HX and 6.00 g of Neosorb 30/60DC in a 150-cc amber bottle. A control blend was prepared by combining 6.48 g of Compound A, 0.75 g of Klucel EXF, 0.90 g of HEC Natrosol 250 HX and 6.72 g of Neosorb 30/60DC in a 150-cc amber bottle. Both the test and control blends were sieved through #16 screens followed by turbula blending for 20 minutes. In each case, 0.15 g of magnesium stearate was added followed by an additional 5 minutes of blending. Tablets were prepared using an F-press with 0.313 X 0.625" caplet tooling to give 775 mg/tablet. Tablets were coated with a solution of 4% (w:w) cellulose acetate 398-10, 2% PEG 3350, 89% acetone and 5% water using a Vector HCT20 LDCS20 Hicoater to a weight gain of 9-10%. Single holes (0.9 mm) were mechanically drilled on the end of the tablets in the band. Dissolution was performed in 900 mL of simulated intestinal fluid (SIN, 50 mM $KH_2PO_4$, pH 6.8) at 37°C using USP Apparatus 2 at 50 rpm. HPLC was used to analyze drug concentrations in solution. Dissolution was performed using a medium consisting of a simulated intestinal fluid (SIN) made using phosphate buffer at pH 6.8 at 37°C using an HPLC potency analysis. The time to deliver 50% of the drug was 10.4 hrs for the SRC control and 8.5 6 hrs for the dispersant test.

Example 14 illustrates the advantage of incorporating sodium bicarbonate into the dosage form.

Example 14

Wet granulations were prepared by combining 169.6 g of [2-(3,4-dichlorophenoxy)-5-fluorobenzyl]-methylamine hydrochloride, 82.9 g of Neosorb P110 (sorbitol, SPI Poyols), and 7.5 g of Klucel EXF (hydroxypropylcellulose, Aqualon) in a 1.7 L bowl for a Procept MI-MI-Pro. The granulation was dry-mixed for 4.0 minutes at an impeller speed of 400 rpm and a chopper speed of 1000 rpm. The mixed material was then wet granulated for 4.5 minutes with an impeller speed of 600 rpm and a chopper speed of 1000 rpm using 56 g of ethanol at a liquid addition rate of 20 g/min. The granulated material was then dried in a convection oven at 40°C for 16 hours. The granulated material was milled using a Fitzpatrick L1A with a 0.030-inch Conidur rasping screen operated at 300 rpm. The final composition of the sodium bicarbonate formulation was generated by blending 37.6 g of the granulated material with the following extra-granular components: 12.5 g of Neosorb 30/60 DC (sorbitol, SPI Pharma, Lewes, Del.), 10.5 g of tartaric acid, 5.0 g of Natrosol 250H (hydroxyethylcellulose, Aqualon), and 3.5 g of sodium bicarbonate. The listed components were bottle-blended in a Turbula mixer for 15 minutes. Magnesium stearate (0.88 g) was then added followed by an additional mixing in the Turbula for 2 minutes. Cores were compressed using a Manesty single-station F-press with an oblong shape (0.3125 x 0.6250 inches, Thomas Engineering) to a tablet core weight of 775 mg and tablet core thickness of 0.281 inches. Cores were coated with a solution of 3.3% (w:w) cellulose acetate 398-10, 1.7% PEG 3350, 90.0% acetone and 5.0% water using a Vector LDCS20 to a weight gain of 7-9% of the mean tablet core weight. One delivery port (1.0 mm) was positioned at one end (band) of the oval tablet by mechanical drilling. In addition, a control formulation without sodium bicarbonate was formulated with identical processing, coating formulation, and delivery port orientation/size. The control tablet core consisted of the following composition: 42.9 g of the granulated material, 14.31 g of Neosorb 30/60 DC (sorbitol, SPI Pharma), 7.0 g of tartaric acid, 4.9 g of Natrosol 250H (hydroxyethylcellulose, Aqualon), and 0.88 g of magnesium stearate. USP Apparatus 2 dissolution at 50 rpm was performed using 900 mL of simulated intestinal fluid (SIN, 50 mM $KH_2PO_4$, pH 6.8). Dissolution sample concentrations of Compound A were determined using HPLC. After 24 hours of dissolution testing, 97% of the drug was released from the tablet containing sodium bicarbonate while only 91% of the drug was released from the control tablet.

Example 15 describes preparation of a preferred tablet formulation and process.

Example 15

To prepare a 2000 g granulation, 2000 mL of 90% isopropyl alcohol and 10% water was first prepared. The following ingredients were added to a 10 L bowl for an SP1 model high shear granulator: 427.3 g of sorbitol, 1125.6 g of [2-(3,4-dichlorophenoxy)-5-fluorobenzyl]-methylamine hydrochloride, 134.9 g of HEC 250 HX and 112.5 g of Klucel EXF. These ingredients were mixed at 400 rpm impeller speed and 1000 rpm chopper speed for 5 min. The impeller speed was then set at 500 rpm and chopper maintained at 1000 rpm while 441.7 mL of isopropanol/water were pumped in at a rate of 100 g/min. Mixing was stopped promptly after the liquid addition. The mixture was tray dried in an oven for 16 hrs at 40°C. The dried granulation was milled using an M5A mill with knives forward (300 rpm with a 0.0315" Conidur rasping plate) over about 4 minutes. The mixture was added to a 4-quart V-blender and mixed for 15 min. This mixture was then transferred to an 8-quart V-blender, and 396.9 g of tartaric acid was added. The mixture was blended for 10 min. At this point, 21.0 g of magnesium stearate were added followed by an additional 5-min. of blending. Tablet cores were prepared on a Killian T-100 rotary tablet press using either 0.265 X 0.490" (for 150 mgA; 340 mg tablet weight) or 0.3175 X 0.635" (for 300 mgA; 674 mg tablet weight) caplet tooling. A coating solution was prepared by dissolving 53.2 g of PEG 3350 in 140 g of water, adding 114.8 g of cellulose acetate, then adding 2492 g of acetone while maintaining stirring. The solution was sprayed using an LDCS-20 pan coater spraying at about 20 g/min (outlet temperature 28-30°C, airflow 30 cfm) until the target 6-7% weight gain based on the mean core tablet weights were achieved. The two sizes were coated separately. Tablets were then dried in a tray using an oven at 40°C for 16 hrs. Dried tablets were either mechanically drilled using a 0.9 mm drill or laser drilled to the same size.

Example 16

The following formulations of sildenafil which are within the scope of the invention were made following the general procedure of Example 2, shown in Table X:

<u>Table X: Formulations</u>

<u>all values are in grams</u>

| Component | New Formulations | | | | | | | |
|---|---|---|---|---|---|---|---|---|
| | 1 | 2 | 3 | 4 | 5 | 6 | 7 | 8 |
| Sildenafil Citrate | | 24.3 | 24.3 | 24.3 | 24.3 | 24.3 | 24.3 | 24.3 |
| Xylisorb 90 | 22.06 | 10.75 | 10.75 | 8.1 | 15.6 | 13.1 | 8.1 | 8.1 |
| HPMC | 1.09 | 1.09 | 1.09 | 2.15 | 2.15 | 2.15 | 2.15 | 2.15 |
| HEC | 2.14 | 2.14 | 2.14 | 5 | 5 | 5 | 5 | 5 |
| Magnesium Stearate | 0.43 | 0.43 | 0.43 | 0.45 | 0.45 | 0.45 | 0.45 | 0.45 |
| Sildenafil citrate (micronised) | | 24.3 | | | | | | |
| Sildenafil mesylate | 24.3 | | | | | | | |
| Ascorbic Acid | | 11.31 | 11.31 | 10.0 | | | | |
| Aspartic Acid | | | | | 2.5 | 5 | | |
| Citric Acid | | | | | | | 10 | |
| Tartaric Acid | | | | | | | | 10 |

**Claims**

1. An osmotic pharmaceutical tablet comprising

    (a) a single-layer compressed core comprising

        (i) a non-ripening drug having a solubility per dose less than about 1 mL$^{-1}$,
        (ii) a hydroxyethylcellulose having a weight-average, molecular weight from about 300,000 to about 2,000,000, and
        (iii) an osmagent,

    wherein said hydroxyethylcellulose is present in said core from about 2.0% to about 20% by weight and said osmagent is present from about 15% to about 75% by weight;
    (b) a water-permeable layer surrounding said core; and (c) at least one passageway within said layer (b) for delivering said drug to a fluid environment surrounding said tablet.

2. The osmotic tablet of Claim 1 wherein said hydroxyethylcellulose has a weight average molecular weight between 700,000 and 1,500,000.

3. The osmotic tablet of Claim 2 wherein said hydroxyethylcellulose is present in said core from about 3% to about 15% by weight.

4. The osmotic tablet of Claim 1 wherein said osmagent present in said core is a sugar.

5. The osmotic tablet of Claim 4 wherein said sugar is sorbitol.

6. The osmotic tablet of Claim 1 wherein the combination of said non-ripening drug and said osmagent have an average ductility from about 100 to about 200 Mpa.

7. The osmotic tablet of Claim 1 wherein the combination of said non-ripening drug and said osmagent have an average tensile strength from about 0.8 to about 2.0 Mpa.

**8.** The osmotic tablet of Claim 1 wherein the combination of said non-ripening drug and said osmagent have an average brittle fracture index less than about 0.2.

**9.** The osmotic tablet of Claim 1, which further comprises a pH modifying agent present in said tablet at between 5 and 25% by weight.

**10.** The osmotic tablet of Claim 19 wherein said pH modifying agent, used in combination with non-ripening basic drugs, is selected from the group consisting of tartaric acid, adipic acid, ascorbic acid, benzoic acid, citric acid, fumaric acid, glutamic acid, malic acid, sorbic acid and toluene sulfonic acid.

**11.** The osmotic tablet of Claim 9 wherein such tablet has a surface Area to volume ratio greater than 0.6 mm$^{-1}$.

**12.** The osmotic tablet of Claim 1 wherein said tablet is in an oblong shape such that the ratio of the major axis to minor axis is between 1.3 and 3.

**13.** The osmotic tablet of Claim 1 wherein said tablet is in a caplet shape such that the ratio of the major axis to minor axis is between 1.3 and 3.

**14.** The osmotic tablet according to Claims 12 or 13, further comprising a single hole formed within 3 mm of intersection of the major axis and the exterior of the tablet.

**15.** The osmotic tablet of claim 1, wherein at least 30% by weight of said tablet core is said non-ripening drug.

**Patentansprüche**

**1.** Osmotisch wirksame pharmazeutische Tablette, die umfasst:

(a) einen einlagigen komprimierten Kern, der

(i) einen nichtreifenden Arzneistoff mit einer Löslichkeit pro Dosis von weniger als etwa 1 ml$^{-1}$
(ii) eine Hydroxyethylcellulose mit einem massegemittelten Molekulargewicht von etwa 300000 bis etwa 2000000 und
(iii) ein Osmagent umfasst,

wobei die Hydroxyethylcellulose in dem Kern in einer Menge von etwa 2,0 bis etwa 20 Gew.-% vorhanden ist und das Osmagent in einer Menge von etwa 15 bis etwa 75 Gew.-% vorhanden ist;
(b) eine den Kern umgebende wasserdurchlässige Schicht und
(c) mindestens eine Passage in der Schicht (b) zur Abgabe des Arzneistoffs an eine die Tablette umgebende fluide Umgebung.

**2.** Osmotisch wirksame Tablette nach Anspruch 1, wobei die Hydroxyethylcellulose ein massegemitteltes Molekulargewicht zwischen 700000 und 1500000 aufweist.

**3.** Osmotisch wirksame Tablette nach Anspruch 2, wobei die Hydroxyethylcellulose in dem Kern in einer Menge von etwa 3 bis etwa 15 Gew.-% vorhanden ist.

**4.** Osmotisch wirksame Tablette nach Anspruch 1, wobei das in dem Kern vorhandene Osmagent ein Zucker ist.

**5.** Osmotisch wirksame Tablette nach Anspruch 4, wobei der Zucker Sorbit ist.

**6.** Osmotisch wirksame Tablette nach Anspruch 1, wobei die Kombination aus dem nichtreifenden Arzneistoff und dem Osmagent eine durchschnittliche Duktilität von etwa 100 bis etwa 200 MPa aufweist.

**7.** Osmotisch wirksame Tablette nach Anspruch 1, wobei die Kombination aus dem nichtreifenden Arzneistoff und dem Osmagent eine durchschnittliche Zugfestigkeit von etwa 0,8 bis etwa 2,0 MPa aufweist.

**8.** Osmotisch wirksame Tablette nach Anspruch 1, wobei die Kombination aus dem nichtreifenden Arzneistoff und dem Osmagent einen durchschnittlichen Sprödbruchindex von weniger als etwa 0,2 aufweist.

9. Osmotisch wirksame Tablette nach Anspruch 1, die ferner ein pH-Modifizierungsmittel, das in der Tablette in einer Menge von zwischen 5 und 25 Gew.-% vorhanden ist, umfasst.

10. Osmotisch wirksame Tablette nach Anspruch 9, wobei das pH-Modifizierungsmittel, das in Kombination mit nichtreifenden basischen Arzneistoffen verwendet wird, aus der Gruppe von Weinsäure, Adipinsäure, Ascorbinsäure, Benzoesäure, Citronensäure, Fumarsäure, Glutaminsäure, Äpfelsäure, Sorbinsäure und Toluolsulfonsäure ausgewählt ist.

11. Osmotisch wirksame Tablette nach Anspruch 9, wobei die Tablette ein Oberfläche/Volumen-Verhältnis von größer als 0,6 mm$^{-1}$ aufweist.

12. Osmotisch wirksame Tablette nach Anspruch 1, wobei die Tablette in einer Oblongform derart, dass das Verhältnis von Hauptachse zu Nebenachse zwischen 1,3 und 3 liegt, vorliegt.

13. Osmotisch wirksame Tablette nach Anspruch 1, wobei die Tablette in einer Capletform derart, dass das Verhältnis von Hauptachse zu Nebenachse zwischen 1,3 und 3 liegt, vorliegt.

14. Osmotisch wirksame Tablette nach Anspruch 12 oder 13, die ferner ein einzelnes Loch, das innerhalb von 3 mm des Schnittpunkts der Hauptachse mit der Außenseite der Tablette ausgebildet ist, umfasst.

15. Osmotisch wirksame Tablette nach Anspruch 1, wobei der nichtreifende Arzneistoff mindestens 30 Gew.-% des Tablettenkerns ausmacht.

**Revendications**

1. Comprimé osmotique pharmaceutique, comprenant :

   (a) un noyau compressé à couche unique, comprenant :

   (i) un médicament n'évoluant pas ayant une solubilité par dose inférieure à environ 1 ml$^{-1}$,
   (ii) une hydroxyéthylcellulose ayant un poids moléculaire moyen en poids compris entre environ 300 000 et environ 2 000 000, et
   (iii) un agent osmotique,

   où ladite hydroxyéthylcellulose est présente dans ledit noyau à raison d'environ 2,0 % à environ 20 % en poids et ledit agent osmotique est présent à raison d'environ 15 % à environ 75 % en poids ;
   (b) une couche perméable à l'eau entourant ledit noyau ; et
   (c) au moins un passage au sein de ladite couche (b) pour libérer ledit médicament dans un environnement fluide entourant ledit comprimé.

2. Comprimé osmotique selon la revendication 1, dans lequel ladite hydroxyéthylcellulose a un poids moléculaire moyen en poids compris entre 700 000 et 1 500 000.

3. Comprimé osmotique selon la revendication 2, dans lequel ladite hydroxyéthylcellulose est présente dans ledit noyau à raison d'environ 3 % à environ 15 % en poids.

4. Comprimé osmotique selon la revendication 1, dans lequel ledit agent osmotique présent dans ledit noyau est un sucre.

5. Comprimé osmotique selon la revendication 4, dans lequel ledit sucre est le sorbitol.

6. Comprimé osmotique selon la revendication 1, dans lequel la combinaison dudit médicament n'évoluant pas et dudit agent osmotique a une ductilité moyenne allant d'environ 100 à environ 200 MPa.

7. Comprimé osmotique selon la revendication 1, dans lequel la combinaison dudit médicament n'évoluant pas et dudit agent osmotique a une résistance à la traction moyenne comprise entre environ 0,8 et environ 2,0 MPa.

8. Comprimé osmotique selon la revendication 1, dans lequel la combinaison dudit médicament n'évoluant pas et dudit agent osmotique a un indice de rupture fragile moyen inférieur à environ 0,2.

9. Comprimé osmotique selon la revendication 1, qui comprend en outre un agent modificateur du pH présent dans ledit comprimé à raison de 5 à 25 % en poids.

10. Comprimé osmotique selon la revendication 9, dans lequel ledit agent modificateur de pH utilisé en combinaison avec des médicaments n'évoluant pas est sélectionné dans le groupe consistant en l'acide tartrique, l'acide adipique, l'acide ascorbique, l'acide benzoïque, l'acide citrique, l'acide fumarique, l'acide glutamique, l'acide malique, l'acide sorbique et l'acide toluènesulfonique.

11. Comprimé osmotique selon la revendication 9, dans lequel ledit comprimé a un rapport superficie/volume supérieur à 0,6 mm$^{-1}$.

12. Comprimé osmotique selon la revendication 1, dans lequel ledit comprimé a une forme oblongue telle que le rapport entre le grand axe et le petit axe est compris entre 1,3 et 3.

13. Comprimé osmotique selon la revendication 1, dans lequel ledit comprimé a une forme de capelet telle que le rapport entre le grand axe et le petit axe est compris entre 1,3 et 3.

14. Comprimé osmotique selon les revendications 12 ou 13, comprenant en outre un unique trou formé dans les 3 mm autour de l'intersection entre le grand axe et l'extérieur du comprimé.

15. Comprimé osmotique selon la revendication 1, dans lequel au moins 30 % en poids dudit noyau de comprimé est formé dudit médicament n'évoluant pas.

**Fig. 1**  3   2   1

**Fig. 2**  2   B   1   A   3

**Fig. 3**  13   11   12

**Fig. 4**  12   D   11   C   13

## *Fig. 5*

## *Fig. 6*

## *Fig. 7*